(19) Europäisches Patentamt
European Patent Office
Office européen des brevets

(11) **EP 4 417 108 A1**

(12) **EUROPEAN PATENT APPLICATION**
published in accordance with Art. 153(4) EPC

(43) Date of publication:
**21.08.2024 Bulletin 2024/34**

(21) Application number: **22897653.6**

(22) Date of filing: **15.11.2022**

(51) International Patent Classification (IPC):
**A61B 5/00** (2006.01)          **A61B 5/021** (2006.01)
**A61B 5/0245** (2006.01)

(52) Cooperative Patent Classification (CPC):
**A61B 5/00; A61B 5/021; A61B 5/024; A61B 5/0245**

(86) International application number:
**PCT/CN2022/131955**

(87) International publication number:
**WO 2023/093570 (01.06.2023 Gazette 2023/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.11.2021 CN 202111437864**

(71) Applicant: **Huawei Technologies Co., Ltd.
Shenzhen, Guangdong 518129 (CN)**

(72) Inventors:
• **ZHENG, Wenhui
  Shenzhen, Guangdong 518129 (CN)**
• **JIN, Junye
  Shenzhen, Guangdong 518129 (CN)**
• **LIN, Zhongwei
  Shenzhen, Guangdong 518129 (CN)**
• **LI, Dong
  Shenzhen, Guangdong 518129 (CN)**

(74) Representative: **MERH-IP Matias Erny Reichl
Hoffmann
Patentanwälte PartG mbB
Paul-Heyse-Strasse 29
80336 München (DE)**

(54) **PROMPTING METHOD AND APPARATUS, ELECTRONIC DEVICE, AND COMPUTER-READABLE STORAGE MEDIUM**

(57) This application is applicable to the field of device control technologies, and provides a prompt method and apparatus, an electronic device, and a computer-readable storage medium. The method includes: obtaining first feature data of a user in response to a measurement operation (S201), where the first feature data includes an activity intensity of the user and/or an air pressure value; determining an abnormality type of the wearable device and/or the user based on the first feature data (S202), where the abnormality type indicates that the wearable device and/or the user are/is currently in a state in which a measurement condition is not met; and outputting prompt information associated with the abnormality type, where the prompt information is used to prompt the user to adjust the current state (S203). According to the provided technical solutions, the user can discover in time that the current state does not meet a measurement requirement, so that an abnormal state is indicated in time, and a case in which a measurement result is inaccurate because a measurement state does not meet the condition is avoided. In this way, a measurement success rate is increased.

FIG. 2

**Description**

This application claims priority to Chinese Patent Application No. 202111437864.6, filed with the China National Intellectual Property Administration on November 27, 2021 and entitled "PROMPT METHOD AND APPARATUS, ELECTRONIC DEVICE, AND COMPUTER-READABLE STORAGE MEDIUM", which is incorporated herein by reference in its entirety.

**TECHNICAL FIELD**

This application relates to the field of device control technologies, and in particular, to a prompt method and apparatus, an electronic device, and a computer-readable storage medium.

**BACKGROUND**

In recent years, as electronic device technologies become increasingly mature, electronic devices gradually develop to be lightweight and miniaturized. In a case in which original functions and performance of the electronic devices are maintained, sizes of the electronic devices become smaller, and various types of wearable devices or wearable devices that integrate a plurality of functions gradually emerge. For example, a smartwatch may integrate a heart rate detection function and/or a blood pressure detection function in addition to a time display function, so that a user can obtain user feature data of the user without using a heart rate meter or a sphygmomanometer. This greatly improves convenience of a measurement operation.

When a measurement task is performed on the user by using the wearable device, for example, in a scenario in which a heart rate of the user is measured or blood pressure of the user is measured, the measurement task may not be accurately completed because the wearable device is worn randomly. However, most users usually cannot determine whether a current wearing state meets a measurement requirement during measurement due to lack of professional knowledge required for completing a measurement task. This greatly increases a case in which a measurement result is inaccurate because the wearing state does not meet the measurement requirement, and reduces reliability of the measurement result.

**SUMMARY**

Embodiments of this application provide a prompt method and apparatus, an electronic device, and a computer-readable storage medium, to resolve a problem that reliability of a measurement result is low because a user cannot determine whether a current wearing state meets a measurement requirement during measurement based on a measurement technology of a wearable device, and a case in which the measurement result is inaccurate because the wearing state does not meet the measurement requirement frequently occurs.

According to a first aspect, an embodiment of this application provides a prompt method, applied to a wearable device, where the wearable device includes an inflatable airbag, and the prompt method includes:

   obtaining first feature data of a user in response to a measurement operation, where the first feature data includes an activity intensity of the user and/or an air pressure value, and the air pressure value includes an air pressure value in the airbag when the user wears the wearable device to measure blood pressure;
   determining an abnormality type of the wearable device and/or the user based on the first feature data, where the abnormality type indicates that the wearable device and/or the user are/is currently in a state in which a measurement condition is not met; and
   outputting prompt information associated with the abnormality type, where the prompt information is used to prompt the user to adjust the current state.

Implementation of this embodiment of this application has the following beneficial effects. When the measurement operation initiated by the user is received, the first feature data of the user is obtained by using the wearable device, and whether the user is currently in a state in which a measurement task can be completed is determined based on the collected first feature data. If the user is currently in a state in which a measurement task cannot be completed, namely, an abnormal state, an abnormality type corresponding to the user and/or the wearable device is determined based on the first feature data, and prompt information corresponding to the abnormality type is generated. The user may adjust the current measurement state based on the output prompt information, so that an adjusted state meets a condition for completing the measurement task, to automatically identify and prompt an abnormality in a measurement process. Compared with an existing wearable device-based measurement technology, in this embodiment, before a measurement result is output, whether the current measurement state meets a measurement condition may be determined based on the collected first feature data. If the current measurement state does not meet the measurement condition, the measurement task is not continued, but prompt information associated with the current measurement state is output. In this way, the user can discover in time that the current state does not meet a measurement requirement, so that an abnormal state is indicated in time, and a case in which a measurement result is inaccurate because a measurement state does not meet the condition is avoided. In this way, a measurement success rate is increased.

In a possible implementation of the first aspect, the obtaining first feature data of a user in response to a measurement operation includes:

controlling the airbag to be inflated in response to the measurement operation, collecting an air pressure value in the airbag in an inflation process, and using the collected air pressure value as the first feature data.

In a possible implementation of the first aspect, the determining an abnormality type of the wearable device and/or the user based on the first feature data includes:

generating an air pressure change curve based on the air pressure value and airbag inflation time corresponding to each air pressure value; and

determining the abnormality type of the wearable device based on the air pressure change curve.

In a possible implementation of the first aspect, the determining the abnormality type of the wearable device based on the air pressure change curve includes:

if a first air pressure value collected at a first moment is less than or equal to a preset air pressure threshold, determining whether a corresponding moment at which a slope of the air pressure change curve increases to a preset slope is later than a second moment; and

if the corresponding moment at which the slope of the air pressure change curve increases to the preset slope is later than the second moment, determining that the wearable device is of a first abnormality type, where the first abnormality type indicates that wearing tightness of the wearable device is in a loose state, and the second moment is later than the first moment.

In a possible implementation of the first aspect, before the outputting prompt information associated with the abnormality type, the method further includes:

determining, based on the corresponding moment at which the slope of the air pressure change curve increases to the preset slope, a quantity of to-be-tightened grids of a strap that need to be adjusted; and

the outputting prompt information associated with the abnormality type includes:

outputting first prompt information, where the first prompt information is used to prompt the user to tighten the strap of the wearable device by a specified quantity of grids, and the specified quantity of grids is the quantity of to-be-tightened grids of the strap.

In a possible implementation of the first aspect, the determining the abnormality type of the wearable device based on the air pressure change curve includes:

if a first air pressure value collected at a first moment is greater than a preset air pressure threshold, determining that the wearable device is of a second abnormality type, where the second abnormality type indicates that wearing tightness of the wearable device is in a tight state.

In a possible implementation of the first aspect, before the outputting prompt information associated with the abnormality type, the method further includes:

determining a corresponding second air pressure value when a slope of the air pressure change curve decreases to a preset slope; and

determining, based on the second air pressure value, a quantity of to-be-loosened grids of a strap that need to be adjusted; and

the outputting prompt information associated with the abnormality type includes:

outputting second prompt information, where the second prompt information is used to prompt the user to loosen the strap of the wearable device by a specified quantity of grids, and the specified quantity of grids is the quantity of to-be-loosened grids of the strap.

In a possible implementation of the first aspect, the determining the abnormality type of the wearable device based on the air pressure change curve includes:

if a slope of the air pressure change curve is less than a preset slope at a preset third moment, determining that the abnormality type of the wearable device is a third abnormality type, where the third abnormality type indicates that the airbag is in an air leakage state.

In a possible implementation of the first aspect, the outputting prompt information associated with the abnormality type includes:

outputting third prompt information, where the third prompt information is used to prompt the user to send the wearable device for repair.

In a possible implementation of the first aspect, the obtaining first feature data of a user in response to a measurement operation includes:

obtaining, by using a moment at which the measurement operation is detected as a start moment, an activity intensity of the user within preset detection duration before the start moment, and using the activity intensity within the preset detection duration as the first feature data.

In a possible implementation of the first aspect, the determining an abnormality type of the wearable device and/or the user based on the first feature data includes:

dividing the preset detection duration into an active time period and an inactive time period based on a preset activity threshold, where an activity intensity in the active time period is greater than or equal to the activity threshold, and an activity intensity in the inactive time period is less than the activity threshold;
accumulating an integral of an activity intensity in each active time period, to determine a total activity amount of the user within the preset detection duration;
determining expected rest duration of the user based on the total activity amount;
determining rested duration of the user based on a type of a time period to which the start moment belongs; and
if the rested duration is less than the expected rest duration, determining that the abnormality type of the user is a fourth abnormality type, where the fourth abnormality type indicates that the user is in a rest insufficient state.

In a possible implementation of the first aspect, before the outputting prompt information associated with the abnormality type, the method further includes:

determining required rest duration of the user based on a time difference between the expected rest duration and the rested duration; and
the outputting prompt information associated with the abnormality type includes:
outputting fourth prompt information, where the fourth prompt information is used to prompt the user with the required rest duration.

In a possible implementation of the first aspect, the wearable device includes a heart rate collection module and an acceleration sensor. The activity intensity is calculated based on a heart rate value obtained by the heart rate collection module and a movement speed determined by the acceleration sensor.

In a possible implementation of the first aspect, after the outputting prompt information associated with the abnormality type, the method further includes:

obtaining second feature data of the user in response to a remeasurement operation that is fed back by the user based on the prompt information; and
if it is determined, based on the second feature data, that statuses of the wearable device and the user both meet the measurement condition, generating a measurement result based on the second feature data.

According to a second aspect, an embodiment of this application provides a prompt apparatus, used in a wearable device, where the wearable device includes an inflatable airbag, and the prompt apparatus includes:

a first feature data obtaining unit, configured to obtain first feature data of a user in response to a measurement operation, where the first feature data includes an activity intensity of the user and/or an air pressure value, and the air pressure value includes an air pressure value in the airbag when the user wears the wearable device to measure blood pressure;
an abnormality type identifying unit, configured to determine an abnormality type of the wearable device and/or the user based on the first feature data, where the abnormality type indicates that the wearable device and/or the user are/is currently in a state in which a measurement condition is not met; and
a prompt information output unit, configured to output prompt information associated with the abnormality type, where the prompt information is used to prompt the user to adjust the current state.

In a possible implementation of the second aspect, the first feature data obtaining unit includes:
an airbag inflation unit, configured to: control the airbag to be inflated in response to the measurement operation, collect an air pressure value in the airbag in an inflation process, and use the collected air pressure value as the first feature data.

In a possible implementation of the second aspect, the abnormality type identifying unit includes:

an air pressure change curve generation unit, configured to generate an air pressure change curve based on the air pressure value and airbag inflation time corresponding to each air pressure value; and
a measurement state classifying unit, configured to determine the abnormality type of the wearable device based on the air pressure change curve.

In a possible implementation of the second aspect, the measurement state classifying unit includes:

a slope rise duration determining unit, configured to: if a first air pressure value collected at a first moment is less than or equal to a preset air pressure threshold, determine whether a corresponding moment at which a slope of the air pressure change curve increases to a preset slope is later than a second moment; and
a looseness abnormality identifying unit, configured to: if the corresponding moment at which the slope of the air pressure change curve increases to the preset slope is later than the second moment, determine that the wearable device is of a first abnormality type, where the first abnormality type indicates that wearing tightness of the wearable device is in a loose state, and the second moment is later than the first moment.

In a possible implementation of the second aspect, the prompt apparatus further includes:
a to-be-tightened grid quantity determining unit, configured to determine, based on the corresponding moment at which the slope of the air pressure change curve increases to the preset slope, a quantity of to-be-tightened grids of a strap that need to be adjusted.

The prompt information output unit includes:
a tightening prompt unit, configured to output first prompt information, where the first prompt information is used to prompt the user to tighten the strap of the wearable device by a specified quantity of grids, and the specified quantity of grids is the quantity of to-be-tightened grids of the strap.

In a possible implementation of the second aspect, the measurement state classifying unit includes:
an over-tightening abnormality identifying unit, configured to: if a first air pressure value collected at a first moment is greater than a preset air pressure threshold, determine that the wearable device is of a second abnormality type, where the second abnormality type indicates that wearing tightness of the wearable device is in a tight state.

In a possible implementation of the second aspect, the prompt apparatus further includes:

a second air pressure value determining unit, configured to determine a corresponding second air pressure value when a slope of the air pressure change curve decreases to a preset slope; and
a to-be-loosened grid quantity determining unit, configured to determine, based on the second air pressure value, a quantity of to-be-loosened grids of a strap that need to be adjusted.

The prompt information output unit includes:
a loosening prompt unit, configured to output second prompt information, where the second prompt information is used to prompt the user to loosen the strap of the wearable device by a specified quantity of grids, and the specified quantity of grids is the quantity of to-be-loosened grids of the strap.

In a possible implementation of the second aspect, the measurement state classifying unit includes:
an air leakage abnormality identifying unit, configured to: if a slope of the air pressure change curve is less than a preset slope at a preset third moment, determine that the abnormality type of the wearable device is a third abnormality type, where the third abnormality type indicates that the airbag is in an air leakage state.

In a possible implementation of the second aspect, the prompt information output unit includes:
a send-for-repair prompt unit, configured to output third prompt information, where the third prompt information is used to prompt the user to send the wearable device for repair.

In a possible implementation of the second aspect, the first feature data obtaining unit includes:
an activity intensity determining unit, configured to: obtain, by using a moment at which the measurement operation is detected as a start moment, an activity intensity of the user within preset detection duration before the start moment, and use the activity intensity within the preset detection duration as the first feature data.

In a possible implementation of the second aspect, the abnormality type identifying unit includes:

an active time period division unit, configured to divide the preset detection duration into an active time period and an inactive time period based on a preset activity threshold, where an activity intensity in the active time period is greater than or equal to the activity threshold, and an activity intensity in the inactive time period is less than the activity threshold;

a total activity amount calculation unit, configured to accumulate an integral of an activity intensity in each active time period, to determine a total activity amount of the user within the preset detection duration;

an expected rest duration calculation unit, configured to determine expected rest duration of the user based on the total activity amount;

a rested duration determining unit, configured to determine rested duration of the user based on a type of a time period to which the start moment belongs; and

a rest abnormality identifying unit, configured to: if the rested duration is less than the expected rest duration, determine that the abnormality type of the user is a fourth abnormality type, where the fourth abnormality type indicates that the user is in a rest insufficient state.

In a possible implementation of the second aspect, the prompt apparatus further includes:
a required rest duration determining unit, configured to determine required rest duration of the user based on a time difference between the expected rest duration and the rested duration.

The prompt information output unit includes:
a rest prompt unit, configured to output fourth prompt information, where the fourth prompt information is used to prompt the user with the required rest duration.

In a possible implementation of the second aspect, the wearable device includes a heart rate collection module and an acceleration sensor. The activity intensity is calculated based on a heart rate value obtained by the heart rate collection module and a movement speed determined by the acceleration sensor.

In a possible implementation of the second aspect, the prompt apparatus further includes:

a remeasurement response unit, configured to obtain second feature data of the user in response to a remeasurement operation that is fed back by the user based on the prompt information; and

a measurement result output unit, configured to: if it is determined, based on the second feature data, that statuses of the wearable device and the user both meet the measurement condition, generate a measurement result based on the second feature data.

According to a third aspect, an embodiment of this application provides an electronic device, including a memory, a processor, and a computer program that is stored in the memory and that can be run on the processor. When executing the computer program, the processor implements the wearable device-based prompt method according to any one of the implementations of the first aspect.

According to a fourth aspect, an embodiment of this application provides a computer-readable storage medium. The computer-readable storage medium stores a computer program, and when the computer program is executed by a processor, the wearable device-based prompt method according to any one of the implementations of the first aspect is implemented.

According to a fifth aspect, an embodiment of this application provides a computer program product. When the computer program product runs on an electronic device, the electronic device is enabled to perform the wearable device-based prompt method according to any one of the implementations of the first aspect.

According to a sixth aspect, an embodiment of this application provides a chip system, including a processor, where the processor is coupled to a memory, and the processor executes a computer program stored in the memory, to implement the wearable device-based prompt method according to any one of the implementations of the first aspect.

It may be understood that, for beneficial effects of the second aspect to the sixth aspect, refer to related descriptions in the first aspect. Details are not described herein again.

**BRIEF DESCRIPTION OF DRAWINGS**

FIG. 1 is a schematic diagram of transition of a blood pressure measurement instrument according to an embodiment of this application;

FIG. 2 is a flowchart of implementing a wearable device-based prompt method according to an embodiment of this application;

FIG. 3 is a schematic flowchart of initiating a measurement operation according to an embodiment of this application;

FIG. 4(a) to FIG. 4(c) are a schematic flowchart of initiating a measurement operation according to another embodiment of this application;

FIG. 5(a) to FIG. 5(f) are a schematic flowchart of initiating a measurement operation according to still another embodiment of this application;

FIG. 6 is a schematic diagram of displaying prompt information according to an embodiment of this application;

FIG. 7(a) to FIG. 7(c) are a schematic diagram of an output manner of prompt information according to an embodiment of this application;

FIG. 8 is a schematic diagram of a remeasurement process according to an embodiment of this application;

FIG. 9 is a schematic diagram of a structure of a wearable device according to an embodiment of this application.

FIG. 10A and FIG. 10B are a schematic flowchart of a wearable device-based prompt method according to an embodiment of this application;

FIG. 11 is a schematic diagram of an air pressure change curve according to an embodiment of this application;

FIG. 12 is an air pressure change curve corresponding to a case in which a strap of a wearable device is worn too tightly according to an embodiment of this application;

FIG. 13 is a schematic diagram of second prompt information according to an embodiment of this application;

FIG. 14 is an air pressure change curve corresponding to a case in which a strap of a wearable device is worn loosely according to an embodiment of this application;

FIG. 15 is a schematic diagram of a response when there are a plurality of abnormalities according to an embodiment of this application;

FIG. 16 is a schematic diagram of first prompt information according to an embodiment of this application;

FIG. 17 is an air pressure change curve corresponding to a case in which an airbag of a wearable device leaks air according to an embodiment of this application;

FIG. 18(a) to FIG. 18(e) are a schematic diagram of third prompt information according to an embodiment of this application;

FIG. 19 is a schematic diagram of a structure of a wearable device according to an embodiment of this application;

FIG. 20 is a schematic flowchart of a wearable device-based prompt method according to an embodiment of this application;

FIG. 21 is a schematic diagram of time period division according to an embodiment of this application;

FIG. 22 is a schematic diagram of outputting prompt information according to an embodiment of this application;

FIG. 23 is a schematic diagram of abnormality type classification according to an embodiment of this application;

FIG. 24 is a block diagram of a structure of a wearable device-based prompt apparatus according to an embodiment of this application;

FIG. 25 is a schematic diagram of a structure of an electronic device according to an embodiment of this application;

FIG. 26 is a block diagram of a software structure of an electronic device according to an embodiment of this application; and

FIG. 27 is a block diagram of a structure of an electronic device according to an embodiment of this application.

## DESCRIPTION OF EMBODIMENTS

In the following description, for illustration instead of limitation, specific details such as a specific system structure and technology are provided, to thoroughly understand embodiments of this application. However, persons skilled in the art should understand that this application may also be implemented in other embodiments without these specific details. In other cases, detailed descriptions of a well-known system, apparatus, circuit, and method are omitted, so as not to obscure the description of this application with unnecessary details.

It should be understood that the term "include" when used in the specification of this application and the appended claims indicates the presence of the described features, entireties, steps, operations, elements, and/or components, but does not exclude the presence or addition of one or more other features, entireties, steps, operations, elements, components, and/or collections thereof.

It should be further understood that the term "and/or" as used in the specification of this application and the appended claims refers to any combination of one or more of associated items and all possible combinations, and includes such combinations.

As used in the specification of this application and the appended claims, the term "if" may be interpreted as "when" or "once" or "in response to determining" or "in response to detecting" depending on the context. Similarly, the phrase "if it is determined" or "if [described condition or event] is detected" may be interpreted, depending on the context, to mean "once determined" or "in response to determining" or "once [described condition or event] is detected" or "in response to detecting [described condition or event]".

In addition, in the description of the specification of this application and the appended claims, the terms "first", "second", "third", and the like are merely used for distinguishing descriptions, but cannot be understood as indicating or implying relative importance.

Reference to "an embodiment" or "some embodiments" described in the specification of this application means that one or more embodiments of this application include a specific feature, structure, or feature described with reference to embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner.

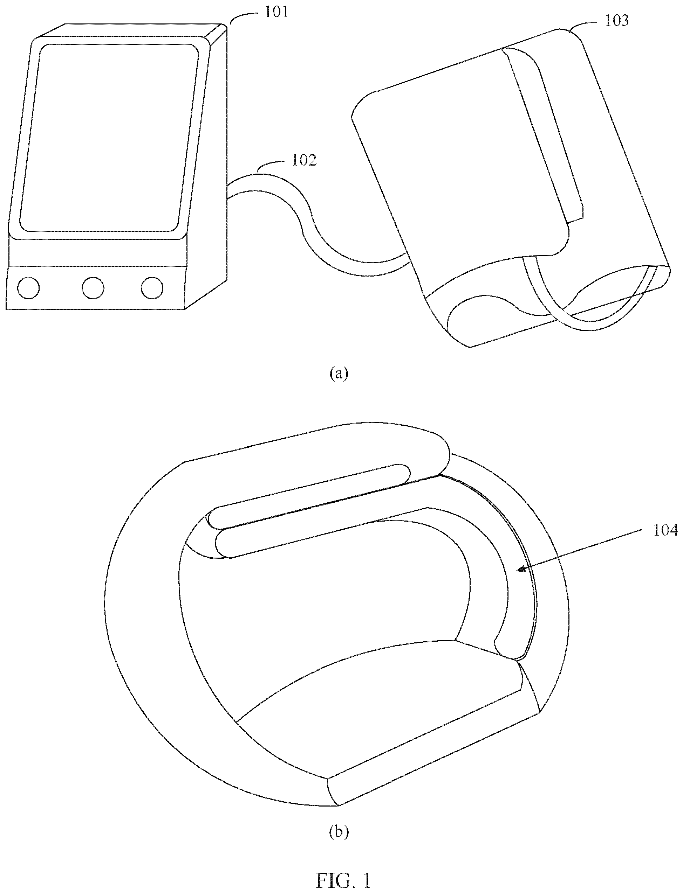

With continuous development of electronic device technologies, more and more devices develop to be lightweight and portable. For example, FIG. 1 is a schematic diagram of transition of a blood pressure measurement instrument according to an embodiment of this application. Herein, (a) in FIG. 1 shows a sphygmomanometer specially used to measure blood pressure of a user. The sphygmomanometer includes at least the following parts: a body device 101, a tube 102 for conveying air during pressurization, and a cuff 103 that includes an inflatable airbag. The cuff 103 is specifically configured to be worn on an arm of the user. In a measurement process, an air pump in the body device 101 may inflate the airbag in the cuff 103 through the tube 102, and obtain an air pressure value that is of the arm of the user and that is fed back in the airbag inflation process. The body device 101 determines blood pressure of the user based on the fed-back air pressure value, and displays a corresponding measurement result on a display module on the body device. It can be learned that the sphygmomanometer has a large size, which is not conducive to carrying. In addition, a plurality of components need to cooperate with each other to complete a measurement task. Consequently, convenience of a measurement operation is low. Therefore, to improve measurement convenience, the sphygmomanometer is smaller in size, and may even be integrated into a wearable device, as shown in (b) in FIG. 1. The wearable device is specifically a smartwatch, and the smartwatch includes an airbag 104. The smartwatch may inflate the airbag 104 by using a built-in micro air pump, to simulate an inflation process of the cuff 103 in the sphygmomanometer. Similarly, the smartwatch may also collect an air pressure value fed back by a wearing area in the inflation process of the airbag 104, to determine blood pressure of the user based on the air pressure value, and complete a measurement task. It can be learned that, compared with the sphygmomanometer, the smartwatch has a significant difference in both size and quantity of components, is improved in portability and reduced in size, and enables the user to complete a measurement task anytime and anywhere.

Although some measurement devices such as a device with a blood pressure measurement function may be integrated into the wearable device to improve measurement convenience, a new problem emerges. Due to flexibility of a wearing manner of the wearable device, in some scenarios, a status of wearing the wearable device by the user does not meet a requirement for performing a measurement operation. Consequently, a measurement result may be inaccurate. For example, during blood pressure measurement, the wearable device is a smartwatch, and a corresponding airbag is disposed in the smartwatch. If the user wears the watch too loosely, that is, a gap between the smartwatch and a skin surface of a wrist of the user is excessively large, in an airbag inflation process, an air pressure value detected at a start phase is less than an expected air pressure value. Similarly, if the user wears the watch too tightly, it indicates that initial pressure exists between the smartwatch and a skin surface of a wrist of the user. In this case, when the airbag is inflated, an air pressure value detected at a start phase is greater than an expected air pressure value. It can be learned that if the status of wearing the smartwatch by the user is not within an appropriate tightness range, a measurement operation cannot be accurately completed. However, because the user usually has no professional knowledge of completing measurement, the user cannot determine how to wear the wearable device to meet a measurement requirement. When the wearable device performs a measurement task, a case in which measurement is inaccurate easily occurs, and consequently, reliability of a measurement result is greatly reduced.

It can be learned that a measurement function (for example, blood pressure measurement or heart rate measurement) is integrated into the wearable device, so that flexibility of the measurement operation is implemented. However, reliability of the measurement result is also easily reduced because a wearing state of the wearable device does not meet a measurement condition. Therefore, a prompt method is urgently required, to prompt the user when it is detected that the wearing state of the wearable device of the user does not meet the measurement condition, and identify an abnormality wearing state, thereby improving reliability of the measurement result.

Embodiment 1

Therefore, to resolve a disadvantage of an existing wearable device-based measurement technology, this application provides a wearable device-based prompt method. The wearable device-based prompt method may be specifically executed by an electronic device. The electronic device may be an electronic device like a smartphone, a tablet computer, a computer, a smartwatch, or a smart band. If the foregoing prompt method is executed by a wearable device like a smartwatch or a smart band, generated prompt information may be displayed by using a display module configured on the wearable device, or prompt information may be broadcast by using a speaker. If the foregoing prompt method is executed by a non-wearable device like a smartphone, a tablet computer, or a computer, prompt information may be displayed by using a display module configured on the non-wearable device in a manner of communicating with the wearable device, or prompt information may be broadcast by using a speaker.

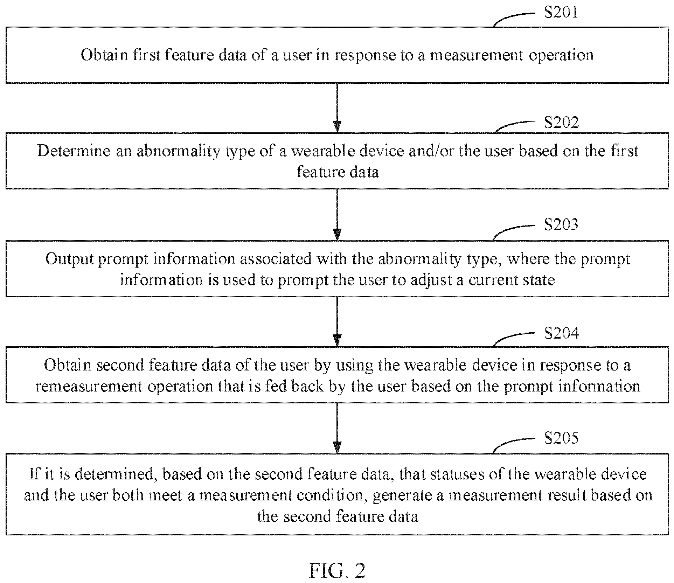

FIG. 2 is a flowchart of implementing a wearable device-based prompt method according to an embodiment of this application. Details are as follows.

S201: Obtain first feature data of a user in response to a measurement operation, where the first feature data includes an activity intensity of the user and/or an air pressure value, and the air pressure value includes an air pressure value in the airbag when the user wears the wearable device to measure blood pressure.

In this embodiment, the electronic device may receive, by using an interaction module, a measurement operation initiated by the user. For example, a touchscreen is configured on the electronic device, and the user may tap a corresponding control on the touchscreen of the electronic device, to initiate the measurement operation to the electronic device.

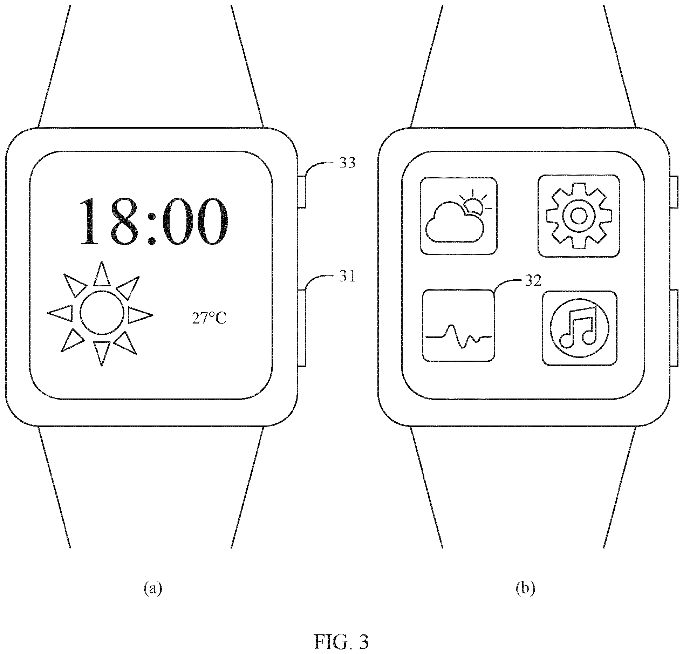

For example, FIG. 3 is a schematic flowchart of initiating a measurement operation according to an embodiment of this application. As shown in FIG. 3, the electronic device is specifically a smartwatch. A home screen of the smartwatch displays current time, and may further display weather information of an area in which the user is located, as shown in (a) in FIG. 3. A function key 31 is configured on the smartwatch. After detecting that the user taps the function key 31, the smartwatch may enter an application menu, and display installed function applications. Certainly, the user may alternatively enter the foregoing application menu based on a preset finger instruction, for example, swiping to the left on a screen, where the application menu is shown in (b) in FIG. 3. The application menu installed on the electronic device includes a plurality of different applications, including an application 32 for performing blood pressure measurement on a user. If the smartwatch detects that the user taps the application 32, it is identified that blood pressure measurement needs to be performed on the user, that is, the foregoing measurement operation is initiated, and step S201 is performed.

In a possible implementation, a shortcut key 33 is further configured on the smartwatch, and the shortcut key 33 may be bound to any application in the application menu in advance. If the shortcut key 33 is bound to the application 32 for blood pressure measurement, the shortcut key 33 is tapped on the home screen, and it is identified that blood pressure measurement needs to be performed on the user, that is, the foregoing measurement operation is initiated, and step S201 is performed.

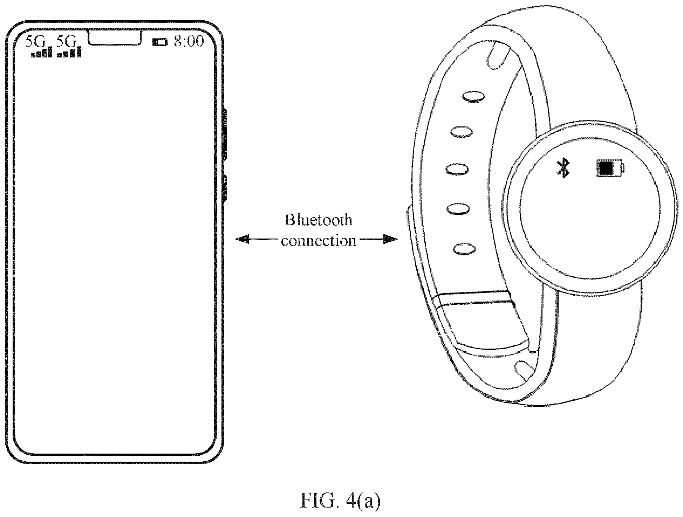

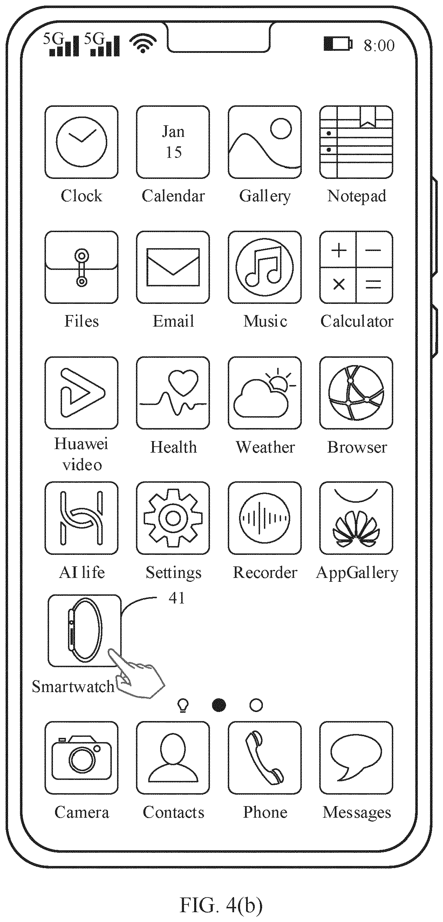

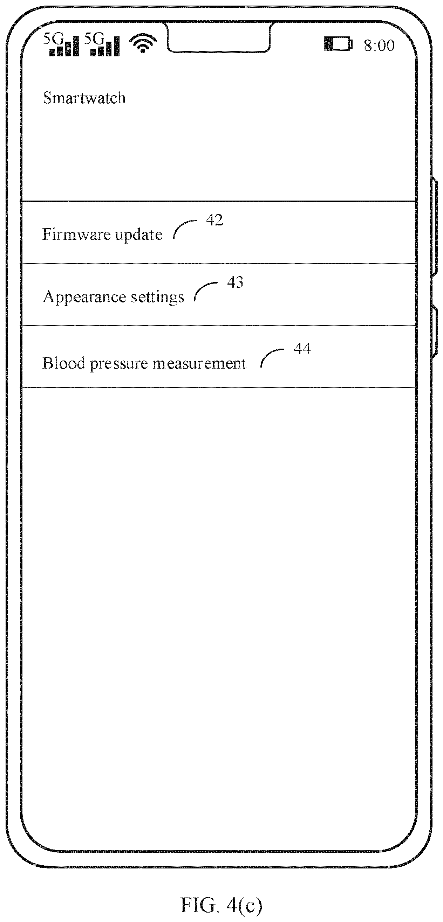

For example, FIG. 4(a) to FIG. 4(c) are a schematic flowchart of initiating a measurement operation according to another embodiment of this application. As shown in FIG. 4(a), the electronic device is specifically a smartphone, and the wearable device is specifically a smartwatch. A Bluetooth wireless connection may be established between the smartphone and the smartwatch by using a Bluetooth module, to implement data exchange. For example, the smartwatch may receive an instruction sent by the smartphone, and perform a corresponding operation in response to the instruction of the smartwatch. Correspondingly, a control application corresponding to the smartwatch may be further installed on the smartphone, and an instruction for controlling the smartwatch is generated in the control application. As shown in FIG. 4(b), an application 41 of the smartwatch is displayed on a home screen of the smartphone. After it is detected that the user taps the application 41, an interface for controlling the smartwatch may be displayed. As shown in FIG. 4(c), the control interface includes a plurality of control items such as an Update control 42 for updating firmware of the smartwatch, an Appearance settings control 43 for changing a watch face, and further includes an extended function of the smartwatch, for example, a Blood pressure measurement control 44. If it is detected that the user taps the Blood pressure measurement control 44, it is identified that the user initiates a measurement operation, and step S201 is performed.

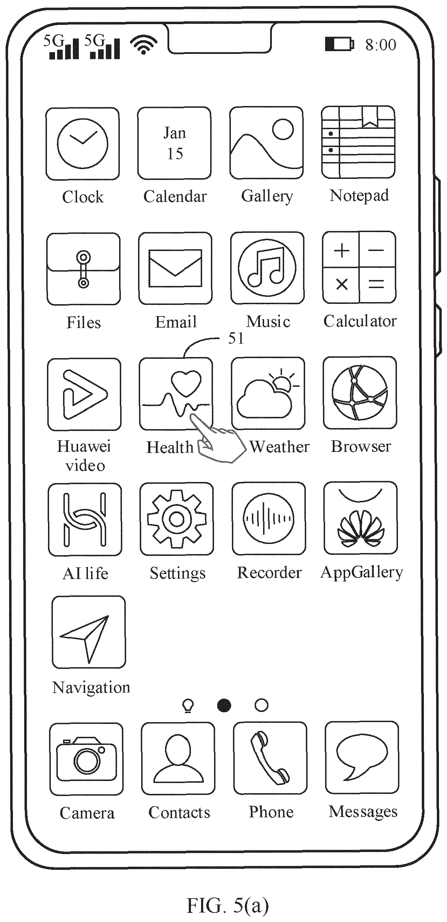

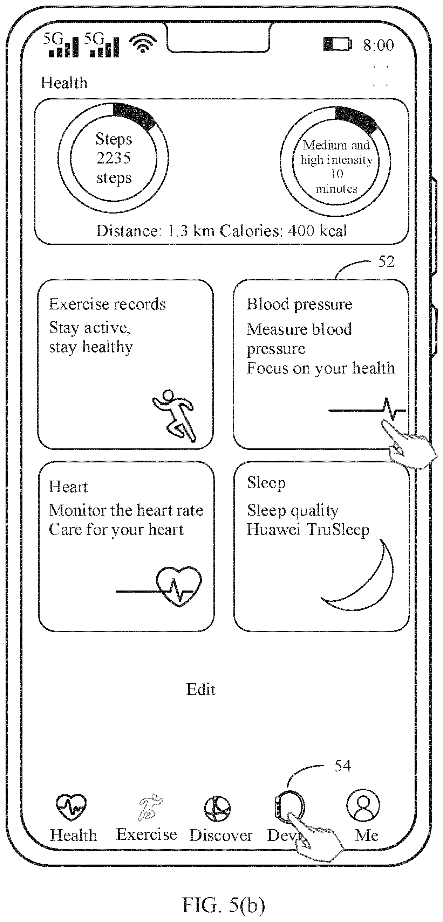

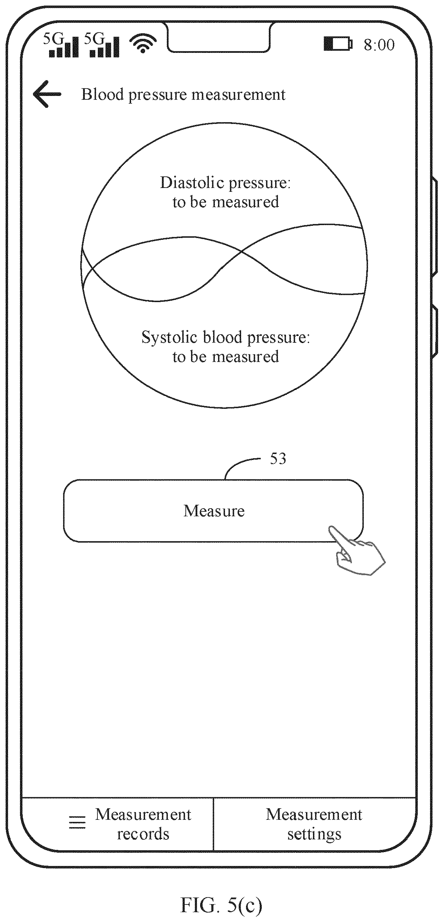

For example, FIG. 5(a) to FIG. 5(f) are a schematic flowchart of initiating a measurement operation according to still another embodiment of this application. An application dedicated to completing measurement, for example, a Health application, like an application 51 shown in FIG. 5(a), may be installed on the smartphone. When the user initiates a measurement task by using the application 51, the user may tap the application 51 to enter a home page of Health, as shown in FIG. 5(b). The home page of Health includes a plurality of function modules, which are an Exercise records module, a Blood pressure module, a Heart module, and a Sleep module. The foregoing modules may be added, deleted, and modified in display location by tapping "Edit", and the like. When blood pressure measurement needs to be performed on the user, the user may tap a widget control 52 corresponding to the Blood pressure module, to enter a blood pressure measurement operation interface in FIG. 5(c). The operation interface includes a Measure control 53. If detecting that the user taps the control 53, the electronic device identifies that the user needs to start blood pressure measurement, sends a measurement instruction to a bound wearable device, and performs an operation of S201.

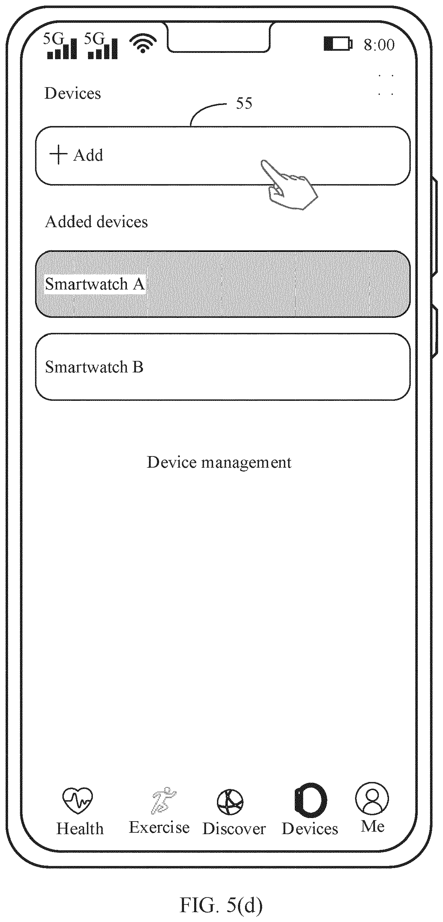

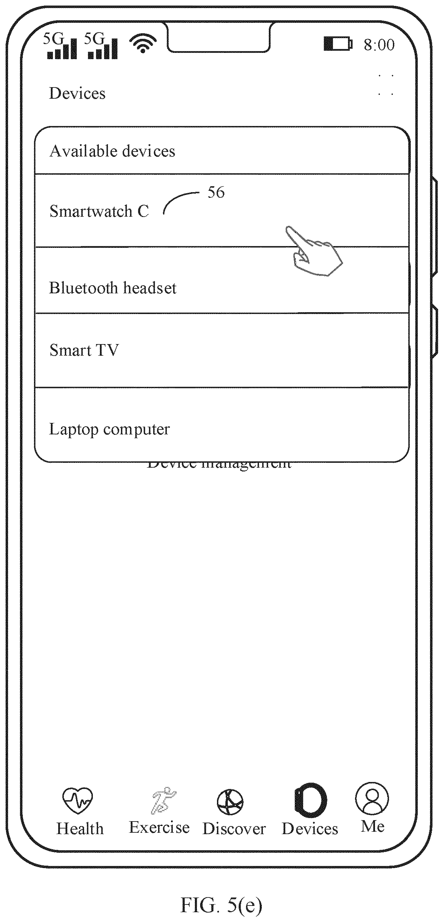

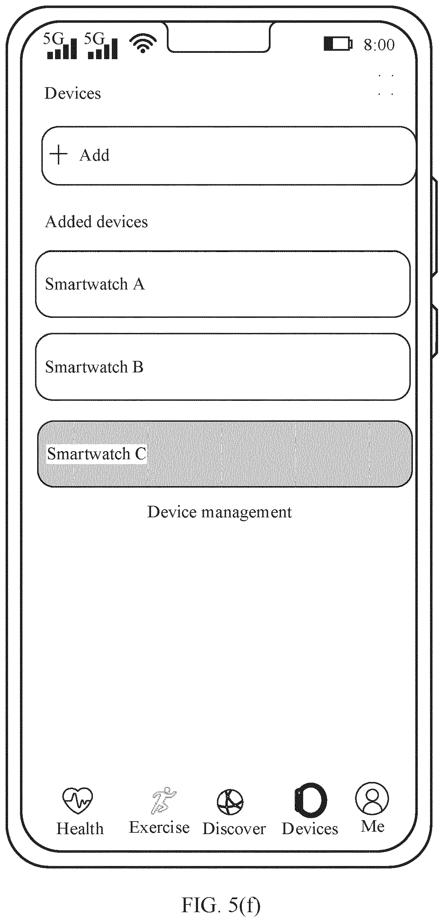

In a possible implementation, if the electronic device is not bound to an associated wearable device, for example, the smartphone is not bound to an associated smartwatch that can perform blood pressure measurement, a device management page may be displayed by tapping a control 54 in FIG. 5(b). The device management page is shown in FIG. 5(d). Bound wearable devices such as a smartwatch A and a smartwatch B are displayed on the page, and the smartwatch A is in a connecting state. When the user needs to add a new wearable device, the user may tap a control 55 for adding a device to add a bound wearable device. In this case, a search page shown in FIG. 5(e) is displayed, and the user may select a corresponding device from connectable devices displayed on the search page. For example, a smartwatch C is a wearable device that can perform blood pressure measurement. In this case, the user taps a control 56 corresponding to the smartwatch C, so that the smartwatch C is added to a list of added devices, and the smartwatch C is set as a device that is being connected, as shown in FIG. 5(f). In this case, the user may return to the home page of Health again, to initiate a blood pressure measurement operation, to perform the initiated measurement operation, and perform step S201.

In a possible implementation, a microphone may be further configured in the electronic device, and the electronic device may start the foregoing measurement process by receiving a voice instruction initiated by the user. For example, the electronic device is specifically a wearable device. The user may say a specified start instruction, for example, "Hi, watch", to enable a voice instruction collection mode of the wearable device. In this case, the wearable device enters the voice instruction collection mode. For example, when "I am here" is fed back by using a speaker, the wearable device may listen to a voice instruction by using a microphone. For example, when the user says "Measure blood pressure", the wearable device identifies that the user initiates a measurement operation. In this case, the wearable device may search a corresponding application from local applications, and starts the corresponding application, to respond to the foregoing measurement operation.

In this embodiment, based on different measurement tasks initiated by the user, first feature data corresponding to the measurement tasks may be collected. For example, if heart rate measurement needs to be performed on the user, the wearable device may obtain an electrocardiosignal, and use the electrocardiosignal as the first feature data. Alternatively, if blood pressure measurement needs to be performed on the user, the wearable device may obtain blood pressure data, and use the blood pressure data as the first feature data. It should be noted that, if the electronic device is a wearable device, the wearable device can directly collect the first feature data of the user, and locally perform the operation of S202 after obtaining the first feature data. If the electronic device is another device other than the wearable device, for example, a smartphone, the another device can send a collection instruction to the wearable device, so that the wearable device can obtain the first feature data of the user. After obtaining the first feature data, the wearable device can feed back the first feature data to the electronic device, and perform the operation of S202.

S202: Determine an abnormality type of the wearable device and/or the user based on the first feature data, where the abnormality type indicates that the wearable device and/or the user are/is currently in a state in which a measurement condition is not met.

In this embodiment, after obtaining the first feature data, the electronic device does not directly generate a corresponding measurement result based on the first feature data, but determines whether the wearable device and/or the user wearing the wearable device meet/meets a preset measurement condition when the first feature data is collected, so that reliability of a measurement process can be ensured. Based on this, the electronic device may determine, based on the first feature data, whether the user and/or the wearable device are/is in an abnormal state. If it is detected that the user and/or the wearable device are/is in a state in which the measurement condition is not met when the first feature data is collected, that is, the user and/or the wearable device are/is in an abnormal state, an abnormality type corresponding to the abnormal state is identified. On the contrary, if it is detected that both the wearable device and the user are in a state in which the measurement condition is met when a user feature is collected, a measurement result may be generated based on the first feature data.

In this embodiment, if the electronic device detects that the user and/or the wearable device are/is in the abnormal state when the first feature data is collected, it indicates that the first feature data collected by the wearable device is inaccurate, the measurement result generated based on the first feature data is unreliable, and the current state does not meet the measurement condition. To help the user determine how to adjust the current state, the electronic device may determine a specific measurement condition that is not met, that is, may determine an abnormality type.

In a possible implementation, the electronic device may store standard feature data corresponding to different abnormality types. The electronic device may calculate a matching degree between the first feature data and the standard feature data corresponding to the different abnormality types. If a matching degree corresponding to any abnormality type is greater than a preset matching degree threshold, it is identified that the current state does not meet a measurement condition, and the abnormality type corresponding to the matching degree that is greater than the matching threshold is used as the abnormality type of the current state. If a matching degree between the first feature data and standard feature data of all abnormality types is less than or equal to a preset matching degree threshold, it is identified that the current state meets all measurement conditions, and a corresponding measurement result may be generated.

In a possible implementation, before S202, the prompt method may further include: If it is determined, based on the first feature data, that statuses of the wearable device and the user both meet the measurement condition, generating a measurement result based on the first feature data.

S203: Output prompt information associated with the abnormality type, where the prompt information is used to prompt the user to adjust the first state.

In this embodiment, different abnormality types correspond to different causes of measurement abnormalities. Measuring a heart rate of the user is used as an example. The wearable device includes a photoplethysmogram (photoplethysmogram, PPG) module that can obtain a PPG signal of the user. If the PPG module is blocked by a stain, a PPG signal fed back based on skin of the user cannot be accurately obtained. In this case, the collected PPG signal is smooth and does not fluctuate, and may be identified as a first abnormality type, and the first abnormality type is associated with a cause of the abnormality, that is, the PPG module is blocked by a stain. If a distance between the skin of the user and the PPG module is long, the wearing state is loose, and strength of the collected PPG signal is low, that is, an amplitude is less than a preset value. In this case, the collected PPG signal may be identified as a second abnormality type, and the second abnormality type is associated with a cause of the loose wearing state. Based on this, after identifying and obtaining an abnormality type corresponding to a first state, the electronic device may output corresponding prompt information based on an abnormality cause corresponding to the abnormality type. The user may determine, based on the foregoing prompt information, a cause of a measurement abnormality, and perform a corresponding adjustment operation.

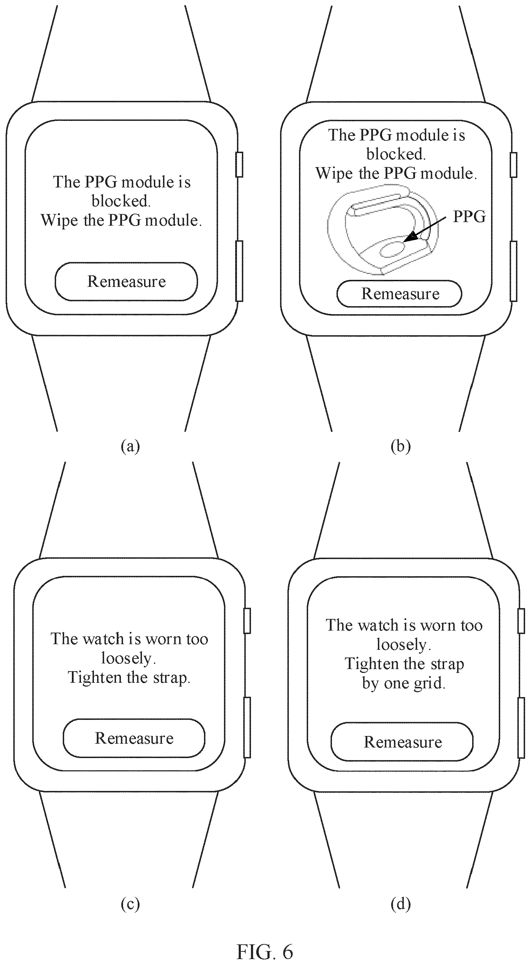

For example, FIG. 6 is a schematic diagram of displaying prompt information according to an embodiment of this application. The foregoing obtaining the heart rate is still used as an example. If the abnormality corresponding to the first abnormality type is caused by stain blocking, prompt information "Wipe the PPG module." may be output, as shown in (a) in FIG. 6. Optionally, to further improve indicativeness and readability of the prompt information, a location corresponding to the PPG module may be indicated in a diagram, as shown in (b) in FIG. 6. If it is detected that the current state is of the second abnormality type, that is, the abnormality is caused by wearing a watch too loosely, prompt information "Tighten the strap." may be output, as shown in (c) in FIG. 6. Optionally, the electronic device may further indicate, to the user, a quantity of to-be-tightened grids of the strap, for example, "Tighten the strap by one grid.", as shown in (d) in FIG. 6.

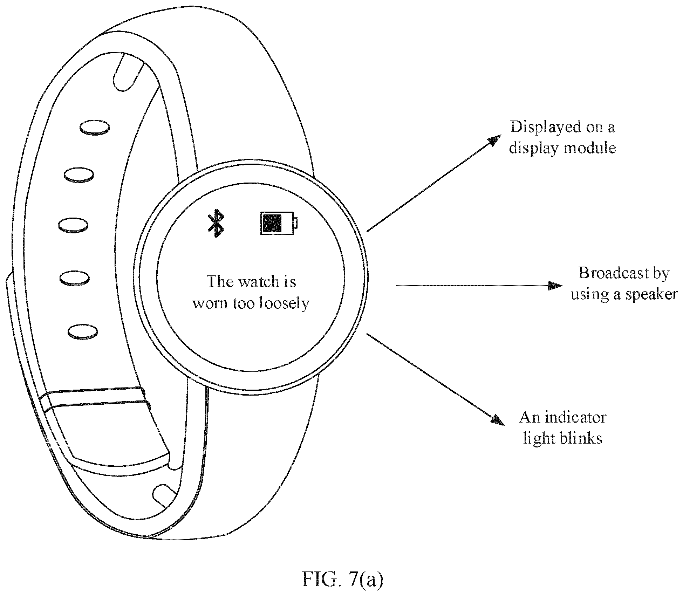

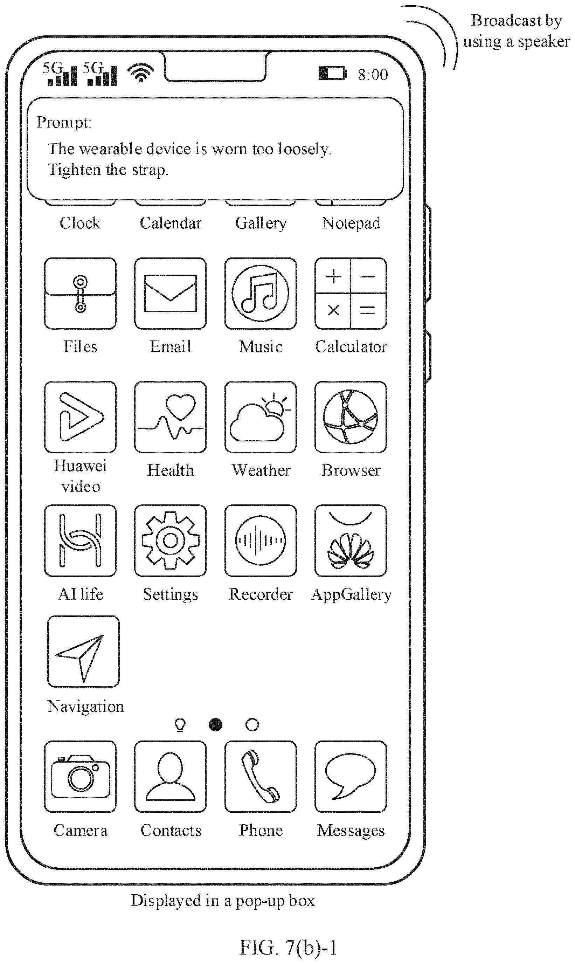

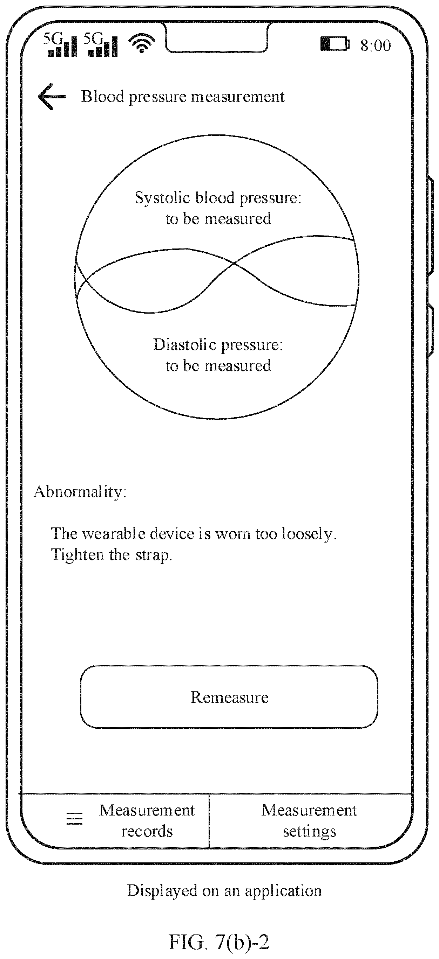

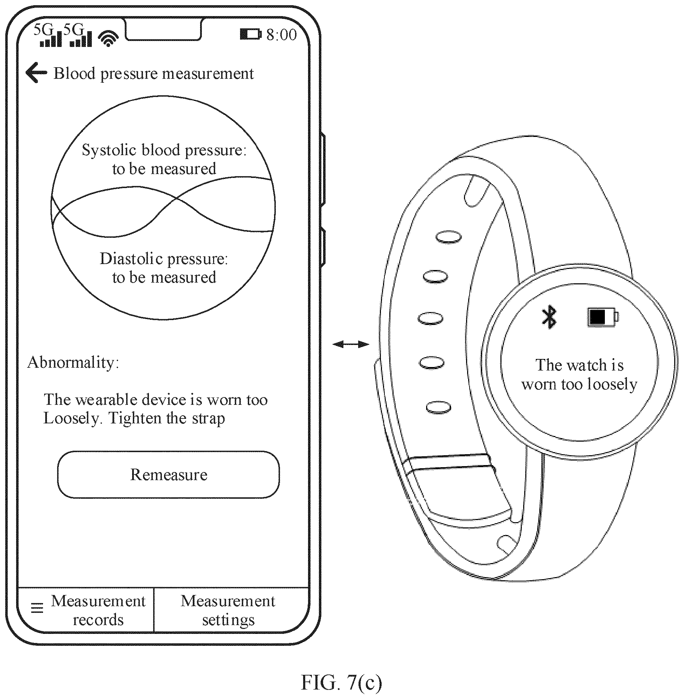

It should be noted that the prompt information may be output in a corresponding manner based on different types of execution bodies. For example, FIG. 7(a) to FIG. 7(c) are a schematic diagram of an output manner of prompt information according to an embodiment of this application. As shown in FIG. 7(a), if the electronic device is a wearable device, the foregoing prompt information may be displayed on a display module of the wearable device, or may be broadcast by using a built-in speaker of the wearable device. If a signal light indicating an abnormality is configured on the wearable device, the user may be prompted with the abnormality in an indicator light blinking manner, so that the user views the prompt information on the wearable device, as shown in FIG. 7(b)-1 and FIG. 7(b)-2. If the electronic device is another device other than a wearable device, the output prompt information may be displayed on a display module of the another device (for example, a smartphone or a tablet computer connected to the wearable device). When being displayed by using the display module, the prompt information may be displayed by using a pop-up box, or may be displayed on a corresponding application interface, or certainly may be displayed in a voice broadcast manner. As shown in FIG. 7(c), the foregoing prompt information may be output on both the wearable device and the foregoing another device, and an output manner may be any one or more of the foregoing manners, which is not limited herein.

Further, in another embodiment of this application, after S203, S204 and S205 may be further performed. Specific descriptions are as follows.

S204: Obtain second feature data of the user in response to a remeasurement operation that is fed back by the user based on the prompt information.

S205: If it is determined, based on the second feature data, that statuses of the wearable device and the user both meet the measurement condition, generate a measurement result based on the second feature data.

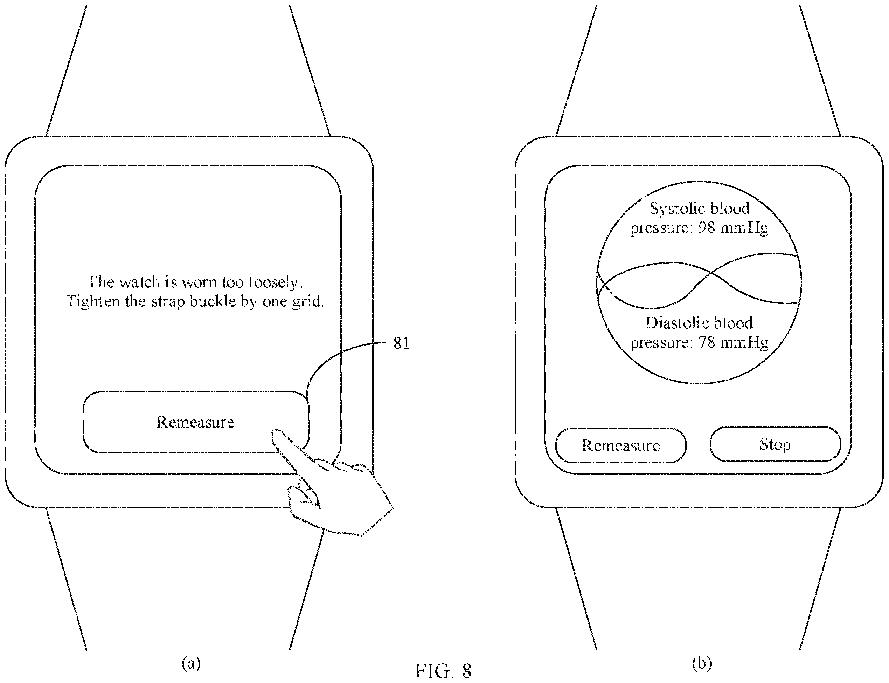

In this embodiment, after adjusting a measurement state based on the prompt information, the user may continue the original measurement task. In this case, the user may initiate a remeasurement operation. For example, FIG. 8 is a schematic diagram of a remeasurement process according to an embodiment of this application. As shown in (a) in FIG. 8, after detecting that the measurement state of the user is an abnormal state, the electronic device may display, on the display module, prompt information corresponding to an abnormality type of the abnormal state, for example, "The strap is worn too loosely. Tighten the strap buckle by one grid." In addition to the prompt information, the prompt interface further includes a Remeasure control 81. After completing adjustment based on the prompt information, the user may tap the Remeasure control 81 to perform a measurement operation again. Based on this, after detecting the Remeasure control 81, the electronic device may identify the measurement operation initiated by the user, and perform step S204. The wearable device re-obtains user feature data of the user, namely, the second feature data, and when detecting that both the statuses of the user and the wearable device meet a measurement condition, generates a corresponding measurement result based on the collected second feature data, as shown in (b) in FIG. 8, and displays a blood pressure value obtained by the user through measurement.

In a possible implementation, after obtaining the second feature data, the electronic device may further determine based on a measurement state corresponding to the second feature data, if it is identified based on the second feature data that the user and/or the wearable device do/does not meet the measurement condition, a corresponding abnormality type again based on the second feature data, and output the prompt information again. For a specific process, refer to the operations in S202 and S203.

In this embodiment of this application, a remeasurement operation is initiated to re-obtain feature data of the user, and when it is detected that the measurement state of the user meets a measurement condition, a corresponding measurement result is generated, so that reliability of the output measurement result is ensured.

It can be learned from the foregoing that, according to the wearable device-based prompt method provided in this embodiment of this application, when the measurement operation initiated by the user is received, the first feature data of the user may be obtained by using the wearable device, and whether the user is currently in a state in which a measurement task can be completed is determined based on the collected first feature data. If the user is currently in a state in which a measurement task cannot be completed, namely, an abnormal state, an abnormality type corresponding to the user and/or the wearable device is determined based on the first feature data, and prompt information corresponding to the abnormality type is generated. The user may adjust the current measurement state based on the output prompt information, so that an adjusted state meets a condition for completing the measurement task, to automatically identify and prompt an abnormality in a measurement process. Compared with an existing wearable device-based measurement technology, in this embodiment, before the measurement result is output, whether the current measurement state meets a measurement condition may be determined based on the collected first feature data. If the current measurement state does not meet the measurement condition, the measurement task is not continued, but prompt information associated with the current measurement state is output. In this way, the user can discover in time that the current state does not meet a measurement requirement, so that an abnormal state is indicated in time, and a case in which a measurement result is inaccurate because a measurement state does not meet the condition is avoided. In this way, a measurement success rate is increased.

Embodiment 2

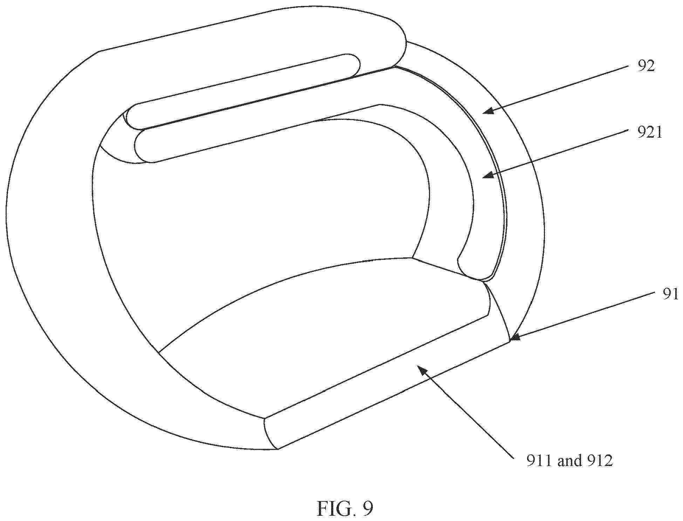

Compared with that in Embodiment 1, a wearable device in this embodiment includes an airbag. The wearable device may inflate the airbag, to obtain an air pressure value fed back by a user in an airbag inflation process, to measure blood pressure of the user. In this case, the first state includes at least the following three abnormality types. For example, FIG. 9 is a schematic diagram of a structure of a wearable device according to an embodiment of this application. As shown FIG. 9, the wearable device is specifically a smartwatch. The smartwatch includes a watch face 91 and a strap 92. An inflatable airbag 921 is disposed on the strap 92. The watch face 91 includes a micro air pump 911 and an air pressure gauge 912. The micro air pump 911 may inflate the airbag 921. The air pressure gauge 912 may collect an air pressure value that is fed back by a skin contact area of the user in an inflation process of the airbag 921, to measure blood pressure of the user.

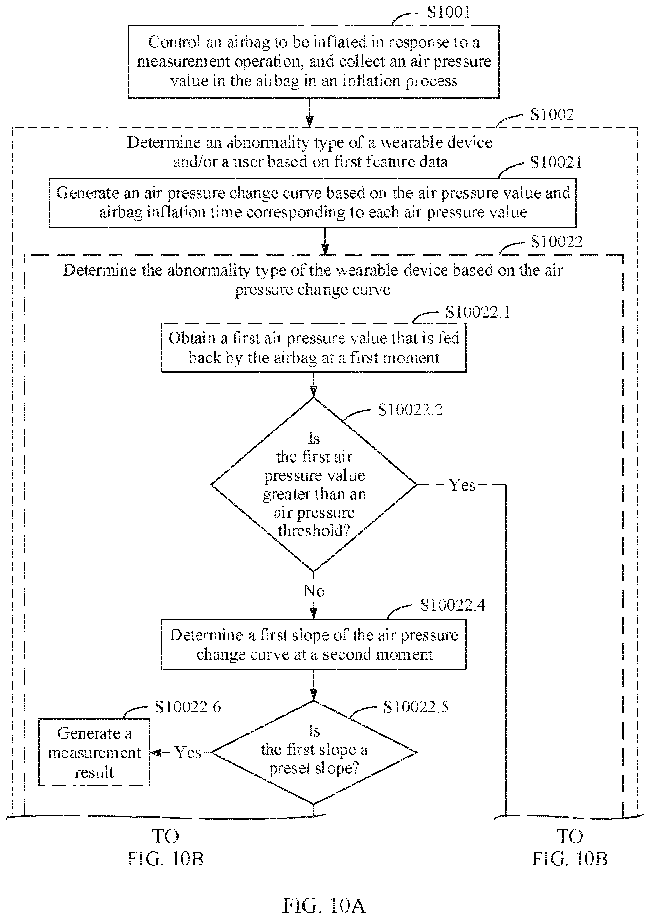

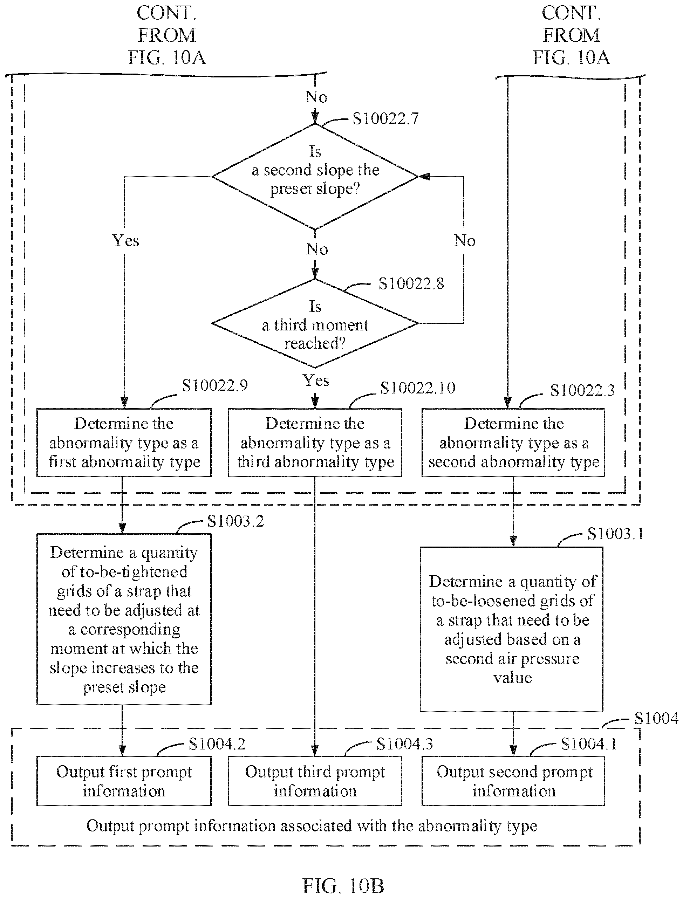

FIG. 10A and FIG. 10B are a schematic flowchart of a wearable device-based prompt method according to an embodiment of this application. As shown in FIG. 10A and FIG. 10B, the method specifically includes the following steps.

S1001: obtaining first feature data of a user in response to a measurement operation includes: controlling the airbag to be inflated in response to the measurement operation, and collecting an air pressure value in the airbag in an inflation process.

A specific implementation of S1001 in this embodiment is the same as that of S201 in Embodiment 1. For a specific description, refer to the related description of S201. Details are not described herein again.

In this embodiment, the first feature data is specifically an air pressure value, and the air pressure value includes an air pressure value in the airbag when the user wears the wearable device to measure blood pressure.

In this embodiment, in a scenario in which blood pressure of the user is measured, the blood pressure of the user needs to be determined by obtaining a pressure change status of the user in a pressure rise process and a pressure change in a pressure drop process. Based on this, in a start phase of measurement, the wearable device may inflate the airbag by using the micro air pump, to determine the pressure change status of the user in the pressure rise process. The wearable device may be configured with a corresponding collection period, and obtain, in the preset collection period, an air pressure value fed back by a part in contact with the wearable device. Therefore, in the entire pressure rise process, the wearable device may obtain a plurality of air pressure values, and use the obtained air pressure values as feature data of the user, namely, the first feature data.

In this embodiment, when a sensor of the wearable device feeds back an air pressure value, the electronic device may further record a time value corresponding to the collected air pressure value, and use the time value as collection time corresponding to the air pressure value. The collection time is used to determine airbag inflation time corresponding to a moment at which the air pressure value is collected. Based on this, the electronic device may associate each air pressure value with corresponding airbag inflation time. If the airbag inflation time is longer, in a normal case, a corresponding collected air pressure value is larger as a volume of the airbag increases due to inflation.

Similarly, this embodiment is specifically executed by an electronic device, and the electronic device may be a wearable device, or may be another device of a non-wearable device. If the electronic device is a wearable device, after obtaining a corresponding air pressure value at each collection moment, the wearable device may store the air pressure value and airbag inflation time corresponding to the air pressure value in an associated manner in a local memory. If the electronic device is another device other than the wearable device, after obtaining the air pressure value and the corresponding airbag inflation time, the wearable device may feed back the air pressure value and the corresponding airbag inflation time to the another device by using a communication connection to the another device, or may first buffer the air pressure value and the corresponding airbag inflation time in a buffer area. When a data feedback condition is met (for example, the pressure rise process ends or a preset feedback moment arrives), all the recorded air pressure values and the corresponding airbag inflation time in the buffer area are sent to the another device, and the another device may perform an operation of S1002 based on the received air pressure values and the corresponding airbag inflation time.

In this embodiment of this application, the airbag of the wearable device is inflated, to obtain the air pressure value fed back by the user in the airbag inflation process, so that blood pressure of the user can be determined. In addition, flexibility and convenience of blood pressure measurement can be improved by configuring the airbag on the wearable device.

S 1002: Determine an abnormality type of the wearable device and/or the user based on the first feature data.

S1004: Output prompt information associated with the abnormality type, where the prompt information is used to prompt the user to adjust the current state.

Specific implementations of S1002 and S1004 are completely the same as those of S202 and S203 in Embodiment 1. For specific descriptions, refer to related descriptions of S202 and S203. Details are not described herein again.

Further, S1002 may specifically include S10021 and S10022, which are specifically described as follows.

S10021: Generate an air pressure change curve based on the plurality of air pressure values and airbag inflation time corresponding to each air pressure value.

S10022: Determine the abnormality type of the wearable device based on the air pressure change curve.

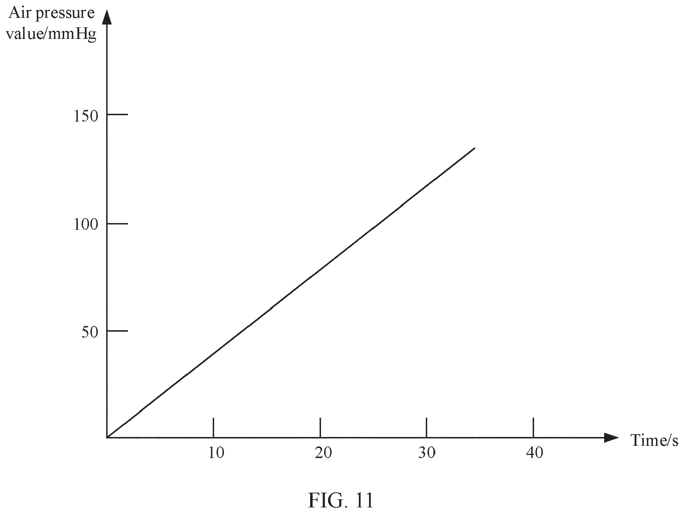

In this embodiment, after collecting the plurality of air pressure values in the airbag, the electronic device may mark, in a preset coordinate system, coordinate points corresponding to the collected air pressure values. A horizontal axis of the coordinate system may be specifically airbag inflation time, and a vertical axis of the coordinate system may be specifically an amplitude of the air pressure value in the airbag. Because each air pressure value is associated with corresponding airbag inflation time, a coordinate point corresponding to the air pressure value may be determined in the foregoing coordinate system, and the coordinate points determined based on the air pressure values are connected accordingly, so that an air pressure change curve corresponding to the current measurement operation can be generated. For example, FIG. 11 is a schematic diagram of an air pressure change curve according to an embodiment of this application. As shown in FIG. 11, a horizontal axis in the air pressure change curve is a time axis, and corresponds to airbag inflation time in a unit of second s. A vertical axis is an amplitude of pressure, and corresponds to a collected air pressure value for a user in an airbag inflation process, and is in a unit of millimeters of mercury mmHg. If statuses of the user and the wearable device both meet a measurement condition of blood pressure measurement, for example, the airbag has no air leakage, and the wearable device is properly worn, a corresponding air pressure change curve is a straight line with a constant slope. As shown in FIG. 11, a collected air pressure value changes with time and increases at a constant speed. Based on this, after generating an air pressure change curve corresponding to the current measurement, the electronic device may analyze the curve, to determine whether the air pressure change curve meets any preset abnormality feature. The electronic device stores curve features corresponding to different abnormality types, namely, the foregoing abnormality features, and compares each abnormality feature with the foregoing air pressure change curve, to implement abnormality identification.

In this embodiment, if the air pressure change curve does not meet all abnormality features, it may be determined that when the first feature data is collected, both the statuses of the user and the wearable device meet the measurement condition. In this case, a corresponding measurement result may be generated. On the contrary, if the air pressure change curve meets any preset abnormality feature, an abnormality type associated with the met abnormality feature is used as an abnormality type of the current state.

The abnormality type specifically includes the following three cases: The strap is worn too loosely. The strap is worn too tightly. The airbag leaks air. Specific implementation processes of the three abnormality types are as follows.

Case 1: The strap is worn too loosely. When the strap of the wearable device is worn too loosely, an abnormality feature shown on the air pressure change curve is that a slope of a start curve segment is less than a preset slope, and duration in which the slope of the air pressure change curve rises to the preset slope is greater than a preset duration threshold, that is, later than a preset second moment.

Case 2: The strap is worn too tightly. When the strap of the wearable device is worn too tightly, an abnormality feature shown on the air pressure change curve is that a slope of a start curve segment is greater than a preset slope. As a result, an air pressure value collected at an initial stage (namely, a preset first moment) of inflating is greater than a preset air pressure threshold.

Case 3: The inflatable airbag on the strap leaks air: When the airbag of the wearable device leaks air, an abnormality feature shown on the air pressure change curve is that a slope of the air pressure change curve is still less than a preset slope at a later stage (namely, a preset third moment) of inflating, that is, there is a smooth curve segment.

In this embodiment, corresponding to the foregoing three different abnormality types, the electronic device may sequentially complete identification of an abnormality feature by using the following steps, which are specifically described as follows.

S10022.1: Collect a corresponding first air pressure value when the airbag is inflated to a first moment.

In this embodiment, the first moment is a moment that is close to a moment at which the airbag starts to inflate. For example, the first moment may be 0.3s or 0.5s after the airbag starts to inflate. At this moment, the wearable device obtains an air pressure value, namely, the first air pressure value, and determines, based on the first air pressure value, whether the airbag has specific initial pressure before being inflated.

S10022.2: Determine whether the first air pressure value is greater than a preset air pressure threshold. If the first air pressure value is greater than a preset air pressure threshold, an operation of S10022.3 is performed. On the contrary, an operation of S10022.4 is performed.

In this embodiment, if it is detected that the first air pressure value is greater than the preset air pressure threshold, it indicates that the airbag has specific initial pressure before being inflated. In this case, it may be determined that the watch of the user is worn too tightly, and the operation of S 10022.3 is performed. If it is detected that the first air pressure value is less than or equal to the air pressure threshold, it indicates that the airbag has small initial pressure before being inflated, and a case in which the watch is not worn too tightly does not appear. It needs to be further determined whether an abnormality like too-loose wearing or airbag leakage exists.

S10022.3: Determine that the abnormality type of the wearable device is a second abnormality type, where the second abnormality type indicates that wearing tightness of the wearable device is in a tight state.

In this embodiment, because the strap is excessively tight, an air pressure value fed back at an initial stage of inflating is greater than a normal value, and a slope of a start curve segment is large in the pressure change curve. The start curve segment specifically refers to a curve segment corresponding to a case in which the airbag starts to being inflated until the slope of the pressure change curve is maintained at the preset slope.

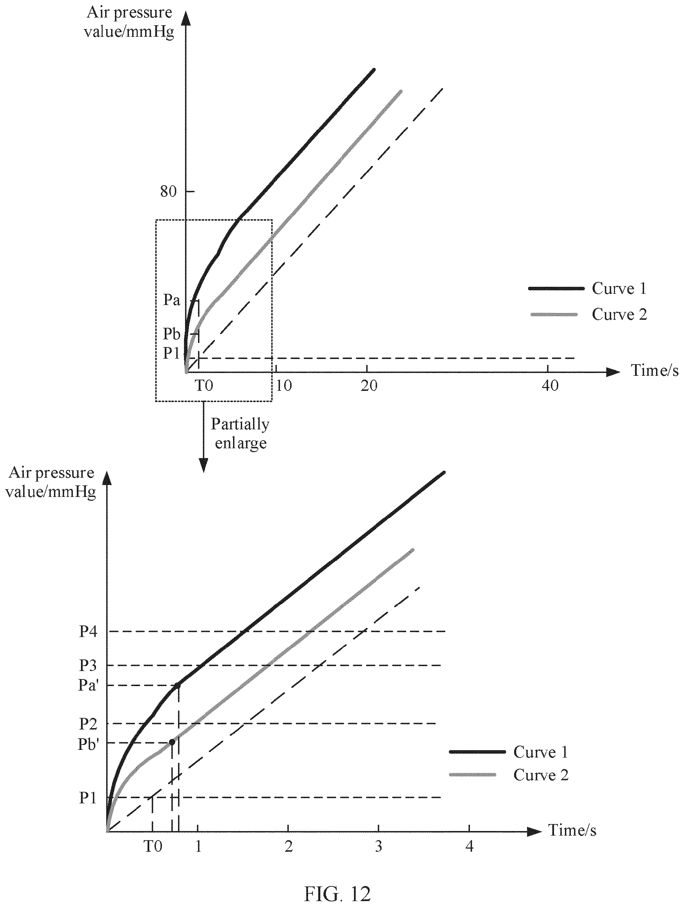

For example, FIG. 12 is an air pressure change curve corresponding to a case in which a strap of a wearable device is worn too tightly according to an embodiment of this application. As shown in FIG. 12, a dashed line represents an air pressure change curve in a normal case, and solid lines (a curve 1 and a curve 2) are air pressure change curves in a case in which the strap is worn too tightly. Regardless of the curve 1 or the curve 2, at a first moment T0, a corresponding air pressure value is greater than an air pressure threshold P1, an air pressure value corresponding to the curve 1 at T0 is Pa, and an air pressure value corresponding to the curve 2 at T0 is Pb, and Pa>Pb>P1. Therefore, both the foregoing two curves belong to air pressure change curves corresponding to the case in which the wearable device is worn too tightly. Refer to the curve 1 and the curve 2. It can be learned that in an initial time period of airbag inflation, air pressure rises quickly, and a slope of a start curve segment of the air pressure change curve is large and gradually decreases, and becomes stable after the slope decreases to a preset slope. This is because the wearable device is worn too tightly, and there is specific initial pressure between the skin of the user and the strap. In this way, when the airbag starts to inflate, large pressure is fed back. Therefore, the air pressure value collected by the wearable device is greater than a normal value, and a slope at the beginning of the curve is also large. In this case, the micro air pump of the wearable device may gradually decrease airbag inflation power, so that the fed-back air pressure value may reach a preset value. Therefore, an increase rate of the air pressure value gradually decreases until the fed-back air pressure value keeps increasing at a constant rate, that is, the slope is remained at the preset slope. Therefore, the electronic device may determine whether the slope of the start curve segment of the air pressure change curve is greater than the preset slope, to determine whether the strap of the wearable device worn by the user is excessively tight.

In a possible implementation, when detecting, at the first moment, that the first air pressure value is greater than the air pressure threshold, the electronic device may stop inflating the airbag, and perform the operation of S1004.

In this embodiment of this application, the first air pressure value obtained in the initial stage of the inflating process is compared with the air pressure threshold, to determine, based on a comparison result, whether the initial pressure exists. If the first air pressure value is greater than the air pressure threshold, the slope of the start curve segment is greater than the preset slope in the air pressure change curve. In this case, the electronic device may determine that the wearable device is worn too tightly, thereby implementing identification of an abnormality type.

Further, after it is determined that the abnormality type of the wearable device is the second abnormality type, before prompt information is generated, the method may further include S1003.1, which is specifically described as follows.

S1003.1: Determine a corresponding second air pressure value when a slope of the air pressure change curve decreases to a preset slope; and determine, based on the second air pressure value, a quantity of to-be-loosened grids of a strap that need to be adjusted.

In a possible implementation, when detecting, at the first moment, that the first air pressure value is greater than the air pressure threshold, the electronic device may continue to inflate the airbag, and monitor a slope of the air pressure change curve. When detecting that the slope of the air pressure change curve is equal to the preset slope, the electronic device stops inflating the airbag, records a current air pressure value, and uses an air pressure value corresponding to a case in which the slope of the air pressure change curve is equal to the preset slope as the second air pressure value. The electronic device may determine, based on the second air pressure value, the quantity of to-be-loosened grids.

In this embodiment, the second air pressure value is specifically an air pressure value corresponding to a case in which it is detected that the slope of the air pressure change curve is equal to the preset slope. When detecting that the strap is worn too tightly, the electronic device needs to prompt the user to loosen the strap. To further improve accuracy of the prompt, the electronic device may further prompt the user with the quantity of to-be-loosened grids. The electronic device may determine, based on the second air pressure value, the quantity of to-be-loosened grids of the strap corresponding to the second air pressure value. A larger second air pressure value indicates that the strap is tightened more tightly, and therefore, a larger quantity of grids of the strap are tightened. On the contrary, a smaller second air pressure value indicates that the strap is not tightened so tightly, and therefore, a smaller quantity of grids of the strap are tightened.

In a possible implementation, the electronic device may store a conversion algorithm between the second air pressure value and the quantity of to-be-loosened grids of the strap, and may import the second air pressure value into the conversion algorithm, to obtain, through calculation, a quantity of to-be-loosened grids of the strap corresponding to the second air pressure value. It should be noted that the calculated quantity of to-be-loosened grids of the strap is a positive integer.

In a possible implementation, the electronic device stores a plurality of air pressure feature values. The air pressure feature values may include an air pressure threshold for comparison at the first moment and an air pressure value used to determine a quantity of to-be-loosened grids. A plurality of air pressure ranges are obtained through division based on the air pressure feature values, and a quantity of to-be-loosened grids of the strap corresponding to the second air pressure value is determined based on an air pressure range within which the second air pressure value falls. For example, the air pressure feature values are respectively P1, P2, P3, P4, and the like, as shown in FIG. 12. P1 may be the air pressure threshold for comparison at the first moment. The plurality of air pressure feature values may be divided into a plurality of air pressure ranges, which are respectively (0, P1), [P1, P2), [P2, P3), [P3, P4), and the like. If the second air pressure value is within (0, P1), it indicates that the wearable device is not worn too tightly. If the second air pressure value is within [P1, P2), a corresponding quantity of to-be-loosened grids of the strap is 1. If the second air pressure value is within [P2, P3), a corresponding quantity of to-be-loosened grids of the strap is 2. If the second air pressure value is within [P3, P4), a corresponding quantity of to-be-loosened grids of the strap is 3, and so on. For example, when a slope of the curve 1 in FIG. 12 is equal to the preset slope, a corresponding air pressure value (namely, the second air pressure value) is Pa', and a value of Pa' is between P2 and P3. Therefore, it may be determined that the strap of the wearable device should be loosened by two grids. For another example, when a slope of the curve 2 in FIG. 12 is equal to the preset slope, a corresponding air pressure value (namely, the second air pressure value) is Pb', and a value of Pb' is between P1 and P2. Therefore, it may be determined that the strap of the wearable device should be loosened by one grid.

Correspondingly, after the quantity of to-be-loosened grids of the strap is determined, S1004 may be specifically S1004.1, which is specifically described as follows.

S1004.1: Output second prompt information, where the second prompt information is used to prompt the user to loosen the strap of the wearable device by a specified quantity of grids, and the specified quantity of grids is the quantity of to-be-loosened grids of the strap.

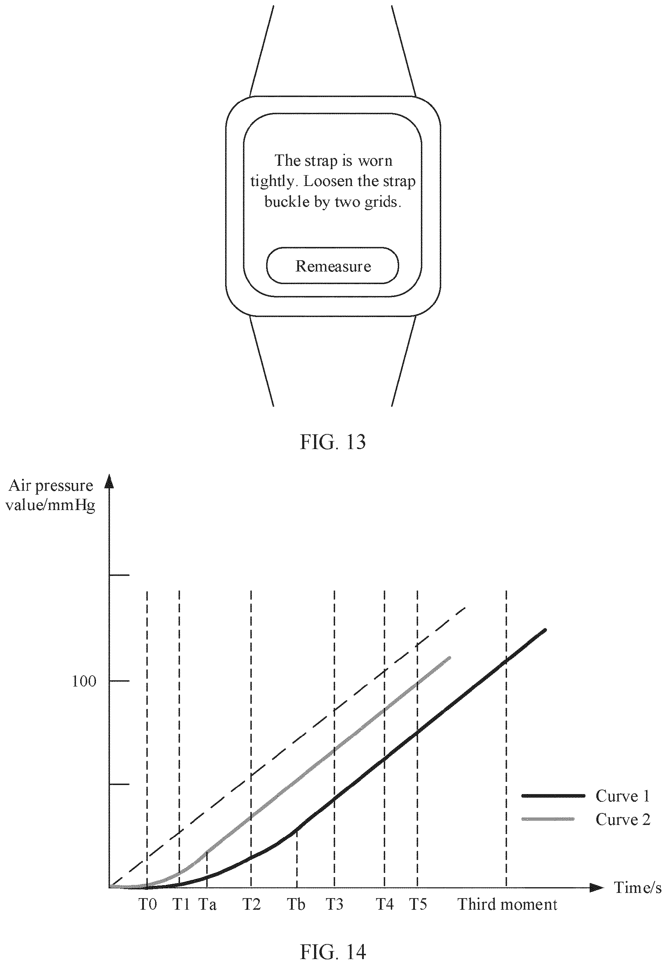

In this embodiment, the electronic device may output the second prompt information that includes the quantity of to-be-loosened grids of the strap, and the user may adjust tightness of the strap based on the second prompt information. For example, FIG. 13 is a schematic diagram of second prompt information according to an embodiment of this application. As shown in FIG. 13, the second prompt information not only prompts the user that the strap needs to be loosened, but also indicates a quantity of to-be-loosened grids, and is displayed as "The strap is worn tightly. Loosen the strap buckle by two grids.".

In a possible implementation, the electronic device may alternatively directly prompt the user to loosen the strap without displaying a quantity of to-be-loosened grids.

In this embodiment of this application, the prompt information including the quantity of to-be-loosened grids is displayed, so that readability of the prompt information can be improved, and the user can adjust a wearing state more accurately. In this way, a case in which a plurality of adjustments are required is reduced, and measurement efficiency is improved.

S10022.4: When it is detected that the first air pressure value is less than or equal to the air pressure threshold, a first slope of the air pressure change curve at the second moment may be determined. The second moment is later than the first moment.

In this embodiment, to determine whether the wearable device is worn too loosely or leaks air, when the airbag is inflated to the preset second moment, the first slope corresponding to the air pressure change curve may be determined, and the first slope is compared with the preset slope. The second moment is a moment after the airbag is inflated for a short period of time (namely, a preset first duration threshold). For example, the second moment may be a moment corresponding to a case in which the airbag is inflated for 2s to 4s.

S10022.5: Determine whether the first slope is the preset slope.

If the first slope is the preset slope, an operation of S10022.6 is performed. If the first slope is not the preset slope, an operation of S 10022.7 is performed.

In this embodiment, if wearing tightness of the user is appropriate, an increase speed of the air pressure of the airbag may be maintained at a preset rate within short inflation time, that is, a corresponding air pressure change curve is maintained at the preset slope within a short time period. Based on this, if it is detected that the first slope of the air pressure change curve at the second moment is already the preset slope, it may be identified that the wearing tightness is appropriate, and an operation of S 10022.6 is performed. On the contrary, the abnormality needs to be further classified and identified, and an operation of S10022.7 is performed.

S10022.6: If the first slope at the second moment is the preset slope, the airbag continues to be inflated for blood pressure measurement, and a measurement result is generated.

In this embodiment, in a case in which tightness is appropriate, a small gap also exists between a skin surface of the user and the strap of the wearable device. In this case, the airbag is inflated for short period of time, and an increase rate of air pressure is low. In other words, when the wearable device is worn normally, a slope may be small and may not reach the preset slope in a first short period of time, but may reach the preset slope after the short period of time. Therefore, if the slope of the air pressure change curve reaches the preset slope before the second moment, it may be identified that the wearable device worn by the user is not in an excessively loose state. In this case, it may be identified that a current state meets a measurement condition, and the airbag may continue to be inflated, to complete a blood pressure measurement operation, and generate a corresponding measurement result.

S10022.7: Obtain a second slope of the air pressure change curve, and determine whether the second slope is the preset slope.

S10022.8: Determine whether a third moment is reached.

In this embodiment, the electronic device periodically obtains an air pressure value fed back in an airbag inflation process, updates an air pressure change curve based on the newly collected air pressure value, and monitors a slope, namely, the second slope, corresponding to a moment at which an updated air pressure value is obtained by collecting an updated air pressure change curve. If the second slope is the preset slope, it indicates that the airbag can be pressurized at the preset slope before the third moment is reached, and it indicates that the airbag does not leak. In this case, an operation of S10022.10 is performed. On the contrary, if the second slope is not the preset slope, before the third moment is reached, the second slope of the air pressure change curve continues to be monitored, and the operation in S10022.7 is returned.

S10022.9: Determine that the abnormality type of the wearable device is a first abnormality type. The first abnormality type indicates that wearing tightness of the wearable device is in a loose state.

In this embodiment, because the strap is excessively loose, the air pressure value fed back at the initial stage of inflating is lower than the normal value, and a slope of the start curve segment in the air pressure change curve is small. It takes a long time to increase the slope to the preset slope, that is, the slope of the air pressure change curve needs to increase to the preset slope at a moment between the second moment and the third moment. In this case, it may be determined that the abnormality type of the wearable device is the first abnormality type. For example, FIG. 14 is an air pressure change curve corresponding to a case in which a strap of a wearable device is worn too loosely according to an embodiment of this application. As shown in FIG. 14, a dashed line represents an air pressure change curve in a normal case, and solid lines (a curve 1 and a curve 2) are air pressure change curves in a case in which the strap is worn loosely. It can be learned that a slope of a start curve segment of the air pressure change curve is small, gradually increases, and becomes stable after being increased to a preset slope. A gap between a skin surface of the user and the wearable device is large, and consequently, when the airbag starts to inflate, the gap between the two is still not filled after a volume of the airbag is expanded. Therefore, an air pressure value obtained by the wearable device at an initial collection stage (before the second moment) is lower than a normal value, and a slope at the beginning of the curve is also small. In this case, the micro air pump of the wearable device may gradually increase airbag inflation power, so that the fed-back air pressure value may reach a preset value. Therefore, an increase rate of the air pressure value gradually increases until the airbag is inflated to a specific extent, and the gap between the skin surface of the user and the wearable device is filled. In this case, the fed-back air pressure value keeps increasing at a constant rate, that is, the slope is remained at the preset slope (after the second moment and before the third moment). In this case, it indicates that the user wears the wearable device too loosely, that is, wearing tightness is in a loose state. In this case, the first state may be identified as a first abnormality type.

In a possible implementation, when detecting that a second slope of the air pressure change curve reaches the preset slope, the electronic device may stop inflating the airbag, and perform the operation of S1003.2 and/or S1004.

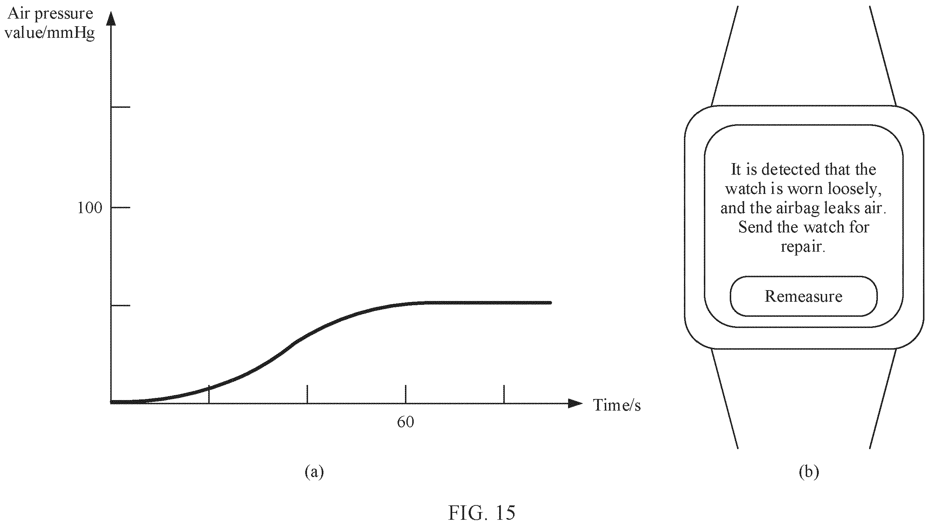

In a possible implementation, when detecting that the second slope of the air pressure change curve reaches the preset slope, the electronic device may continue to inflate the airbag, and monitor a slope of the air pressure change curve. When detecting that the slope of the air pressure change curve is less than the preset slope, the electronic device stops inflating the airbag. In this case, it indicates that not only the strap worn by the user is too loose, but also the airbag leaks. FIG. 15 is a schematic diagram of a response when there are a plurality of abnormalities according to an embodiment of this application. As shown in (a) in FIG. 15, a slope of the air pressure change curve is less than the preset slope for a long time at an initial stage, and is reduced to be less than the preset slope again at a later stage. In this case, the electronic device may determine that the wearable device may correspond to two abnormality types, namely, the first abnormality type and a third abnormality type.

In this embodiment of this application, the slope of the start curve segment of the air pressure change curve is compared with the preset slope. When it is detected that the slope is less than the preset slope and lasts for a long time, it may be determined that the wearable device is worn too loosely, and an abnormality type is identified.

Further, after it is determined that the abnormality type of the wearable device is the first abnormality type, before prompt information is generated, the method may further include S1003.2, which is specifically described as follows.

S1003.2: Determine a quantity of to-be-tightened grids of a strap that need to be adjusted based on a corresponding moment at which the slope of the air pressure change curve increases to the preset slope.

In this embodiment, when detecting that the strap is worn too loosely, the electronic device needs to prompt the user to tighten the strap. To further improve accuracy of the prompt, the electronic device may further prompt the user with the quantity of to-be-tightened grids. The electronic device may determine a corresponding quantity of to-be-tightened grids of the strap based on a corresponding moment at which the slope of the air pressure change curve increases to the preset slope. If the corresponding moment at which the slope of the air pressure change curve increases to the preset slope is later, it indicates that a gap between skin of the user and the strap is larger, and therefore, the quantity of to-be-tightened grids of the strap is larger. On the contrary, if the slope of the air pressure change curve increases to the preset slope at an earlier corresponding moment, it indicates that a gap between skin of the user and the strap is smaller, and therefore, the quantity of to-be-tightened grids of the strap is smaller.

In a possible implementation, the electronic device may store a conversion algorithm between the corresponding moment at which the slope of the air pressure change curve increases to the preset slope and the quantity of to-be-tightened grids of the strap, and may import the corresponding moment at which the slope of the air pressure change curve increases to the preset slope into the conversion algorithm, to obtain, through calculation, a quantity of to-be-tightened grids of the strap corresponding to the moment at which the slope of the air pressure change curve increases to the preset slope. It should be noted that the calculated quantity of to-be-tightened grids of the strap is a positive integer.

In a possible implementation, the electronic device stores a plurality of preset moment values, obtains a plurality of time intervals through division based on the preset moment values, and determines, based on an air pressure time interval within which the moment at which the slope of the air pressure change curve increases to the preset slope falls, a quantity of to-be-tightened grids of the strap corresponding to the moment at which the slope of the air pressure change curve increases to the preset slope. For example, the preset moment values are respectively: T1, T2, T3, T4, and the like, as shown in FIG. 14. T1 may be the foregoing second moment, and the foregoing plurality of preset moment values may be divided into a plurality of duration intervals, which are respectively (0, T1), [T1, T2), [T2, T3), [T3, T4), and the like. If a corresponding moment at which the slope of the air pressure change curve increases to the preset slope is within (0, T1), a case in which the wearable device is not worn too loosely is identified. If a corresponding moment at which the slope of the air pressure change curve increases to the preset slope is within [T1, T2), a corresponding quantity of to-be-tightened grids of the strap is 1. If a corresponding moment at which the slope of the air pressure change curve increases to the preset slope is within [T2, T3), a corresponding quantity of to-be-tightened grids of the strap is 2. If a corresponding moment at which the slope of the air pressure change curve increases to the preset slope is within [T3, T4), a corresponding quantity of to-be-tightened grids of the strap is 3, and so on. For example, for the curve 1 in FIG. 14, a corresponding moment at which the slope of the air pressure change curve increases to the preset slope is Tb, and Tb is between T2 and T3. Therefore, it may be determined that the strap of the wearable device should be tightened by two grids. For another example, for the curve 2 in FIG. 14, a corresponding moment at which the slope of the air pressure change curve increases to the preset slope is Ta, and Ta is between T1 and T2. Therefore, it may be determined that the strap of the wearable device should be tightened by one grid.

Correspondingly, after the quantity of to-be-loosened grids of the strap is determined, S1004 may be specifically S 1004.2, which is specifically described as follows.

S1004.2: Output first prompt information, where the first prompt information is used to prompt the user to tighten the strap of the wearable device by a specified quantity of grids, and the specified quantity of grids is the quantity of to-be-tightened grids of the strap.

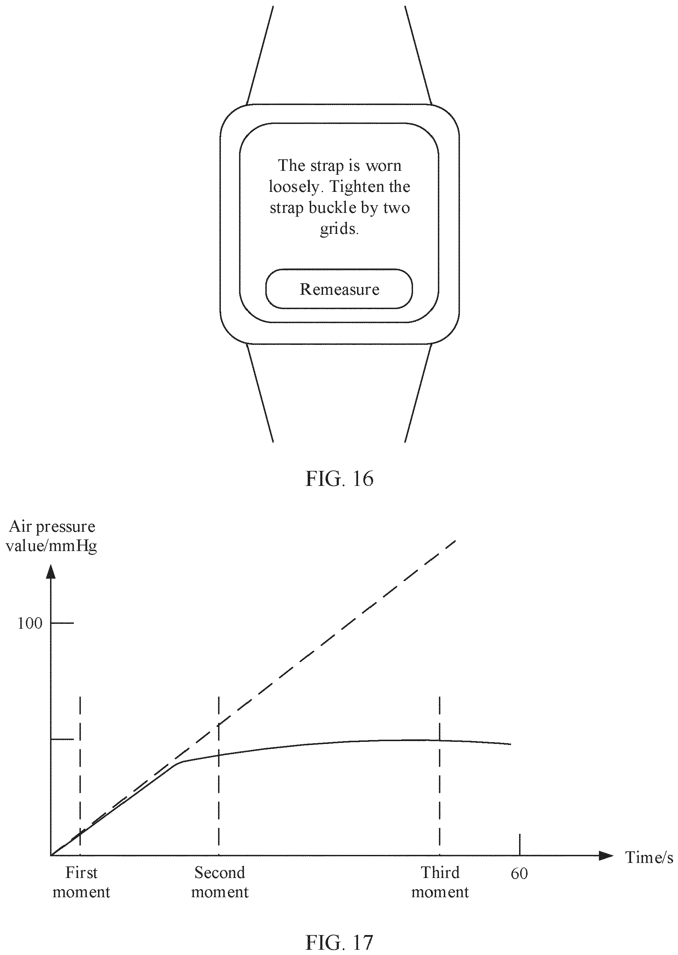

In this embodiment, the electronic device may output the first prompt information that includes the quantity of to-be-tightened grids of the strap, and the user may adjust tightness of the strap based on the first prompt information. For example, FIG. 16 is a schematic diagram of first prompt information according to an embodiment of this application. As shown in FIG. 16, the first prompt information not only prompts the user that the strap needs to be tightened, but also indicates a quantity of to-be-tightened grids, and is displayed as "The strap is worn loosely. Tighten the strap buckle by two grids.".

In a possible implementation, the electronic device may alternatively directly prompt the user to tighten the strap without displaying the quantity of to-be-tightened grids.

In this embodiment of this application, the prompt information including the quantity of to-be-tightened grids is displayed, so that readability of the prompt information can be improved, and the user can adjust a wearing state more accurately. In this way, a case in which a plurality of adjustments are required is reduced, and measurement efficiency is improved.

S10022. 10: If the second slope of the air pressure change curve is still less than the preset slope at the third moment, it is determined that the abnormality type of the wearable device is a third abnormality type.

In this embodiment, the third moment may be a long period of time, for example, any time between 30s and 60s. When the airbag has no abnormality, even if the watch is worn very loosely, the air pressure change curve may reach the preset slope before the third moment, in other words, an air pressure increase rate is constant. However, in a case in which the airbag leaks air, the airbag cannot reach a preset target pressure value (namely, an expected maximum value in a pressure rise process), because the fed-back air pressure value does not reach the foregoing pressure target value, the micro air pump still continuously inflates the airbag, and a slope of the air pressure change curve at the third moment is still less than the preset slope. Therefore, a smooth curve segment, namely, a curve segment whose slope is less than the preset slope, exists in the air pressure change curve. For example, FIG. 17 is an air pressure change curve corresponding to a case in which an airbag of a wearable device leaks air according to an embodiment of this application. As shown in FIG. 17, a dashed line represents an air pressure change curve in a normal case, and a solid line is an air pressure change curve in a case of airbag leakage. It can be learned that a slope of the air pressure change curve is always less than a preset slope, and a slope of a second half of the curve tends to be flat. In this case, it is determined that the airbag of the wearable device worn by the user leaks air, and it is determined that an abnormality type of the wearable device is a third abnormality type.

In this embodiment of this application, it is identified whether there is a smooth curve segment in the air pressure change curve, to determine whether the airbag of the wearable device leaks air, an abnormality type is identified.

Further, when it is detected that the abnormality type of the first state is the third abnormality type, S1004 may be specifically S 1004.3, which is specifically described as follows.

S1004.3: Output third prompt information, where the third prompt information is used to prompt the user to send the wearable device for repair.

In this embodiment, because the airbag of the wearable device leaks air, the user cannot repair the abnormality, and needs to hand over the airbag to a professional for repair or replacement. In this case, the electronic device prompts the user to send the wearable device for repair, and generates the third prompt information for sending the wearable device for repair.

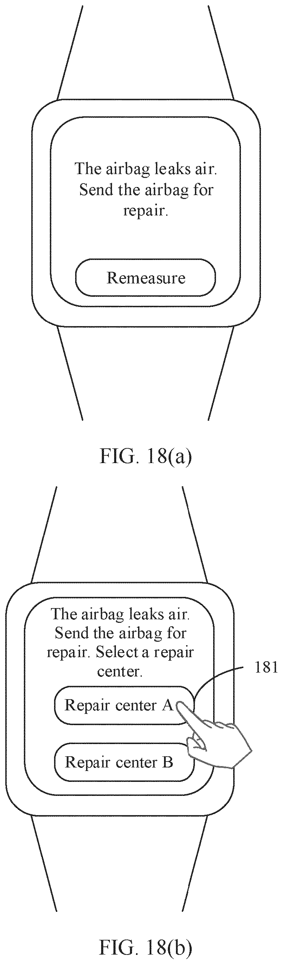

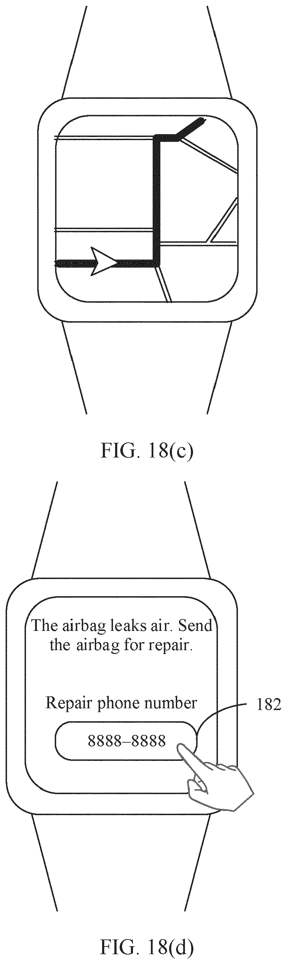

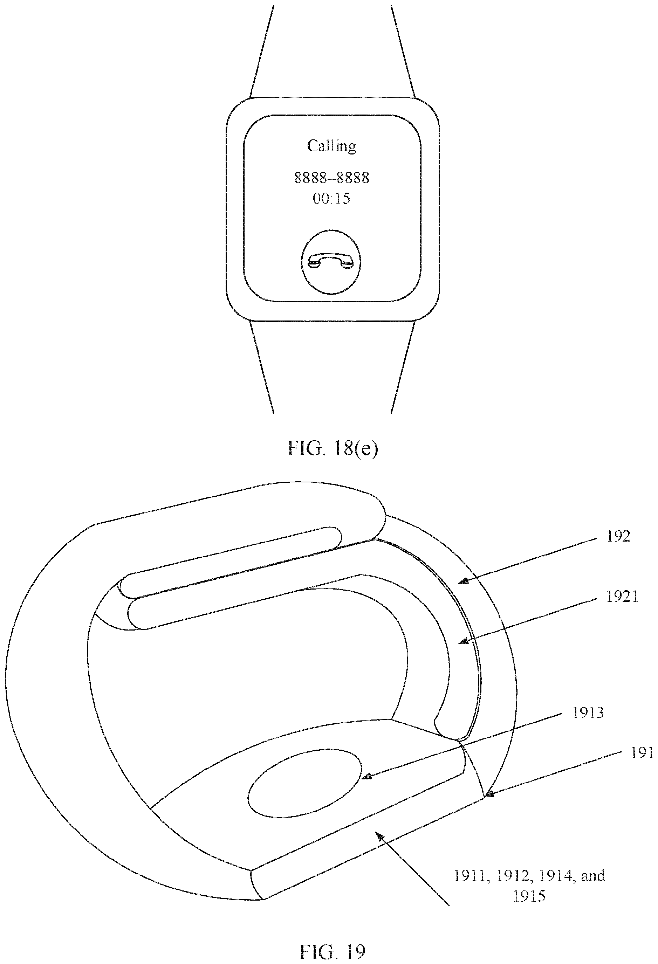

For example, FIG. 18(a) to FIG. 18(e) are a schematic diagram of third prompt information according to an embodiment of this application. As shown in FIG. 18(a), the third prompt information is displayed as "The airbag leaks air. Send the airbag for repair.". Further, to improve guidance of the prompt information for the user and reduce user operations, the third prompt information may further display a navigation jump link of a repair center, as shown in FIG. 18(b). The user may click the navigation jump link to initiate a navigation operation for a specific repair center. For example, if the user clicks a "Repair center A" control 181, a navigation interface for navigation to the repair center A is generated, as shown in FIG. 18(c). Optionally, in addition to displaying the repair center, a repair phone number may be further displayed, as shown in FIG. 18(d). A control 182 for displaying the repair phone number "8888-8888" is displayed. The user may directly dial the repair phone number by tapping the control 182, to send the wearable device for repair, as shown in FIG. 18(e).

In this embodiment of this application, when air leakage of the airbag is detected, prompt information for sending the device for repair is generated, so that the user can determine how to perform processing when an abnormality of air leakage of the airbag occurs.

It should be noted that the air pressure change curve may correspond to one or more abnormality types, that is, the air pressure change curve matches one or more abnormality features. In this case, the prompt information output by the electronic device is determined based on a plurality of abnormality types. As shown in (b) in FIG. 15, the foregoing prompt information may be displayed as "It is detected that the watch is worn loosely, and the airbag leaks air. Send the watch for repair".

Embodiment 3

Compared with that in Embodiment 1, the prompt method in this embodiment of this application is applied to a blood pressure measurement scenario. When blood pressure measurement is performed, the user needs to be in a rest state, in other words, immediate blood pressure measurement after intense exercise is inappropriate. When performing blood pressure measurement, the electronic device may first determine whether the user has performed intense exercise and is still in an active state. Alternatively, the electronic device may determine whether the user has rested for a period of time after intense exercise, and is restored from the active state to the rest state. If the user is in the foregoing active state, or rest time after the exercise is insufficient, a blood pressure measurement condition is not met, and this corresponds to a fourth abnormality type.

Based on this, a wearable device in this embodiment includes a heart rate collection module and an acceleration sensor. The heart rate collection module may be specifically a PPG module. The wearable device may obtain a heart rate value of the user by using the heart rate collection module, and the acceleration sensor may determine a movement speed of the user. For example, FIG. 19 is a schematic diagram of a structure of a wearable device according to an embodiment of this application. As shown in FIG. 19, the wearable device is specifically a smartwatch. The smartwatch includes a watch face 191 and a strap 192. An inflatable airbag 1921 is disposed on the strap 192. The watch face 191 includes a micro air pump 1911, an air pressure gauge 1912, a heart rate collection module 1913, an acceleration sensor 1914, and a gyroscope 1915. The micro air pump 1911 may inflate the airbag 1921. The air pressure gauge 1912 may collect an air pressure value that is fed back by a skin contact area of the user in an inflation process of the airbag 1921, to complete an objective of measuring blood pressure of the user. The heart rate collection module 1913 may determine a heart rate value of the user. The acceleration sensor 1914 and the gyroscope 1915 are configured to determine a movement speed of the user.

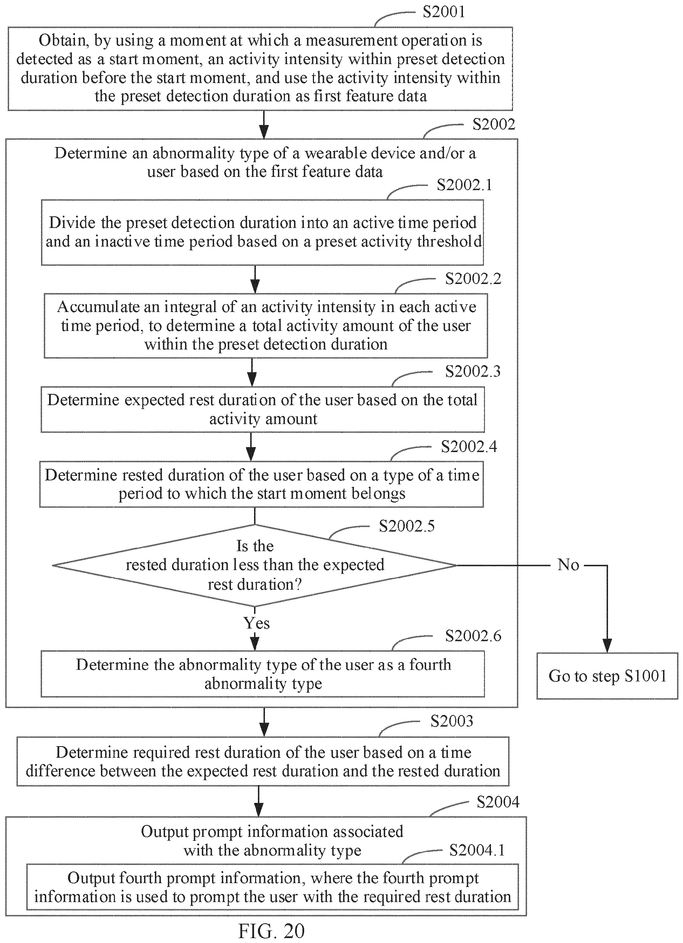

FIG. 20 is a schematic flowchart of a wearable device-based prompt method according to an embodiment of this application. As shown in FIG. 20, the method specifically includes the following steps.

S2001: That a wearable device obtains first feature data of a user in response to a measurement operation includes: obtaining, by using a moment at which the measurement operation is detected as a start moment, an activity intensity of the user within preset detection duration before the start moment, and using the activity intensity within the preset detection duration as the first feature data.

In this embodiment, when responding to the measurement operation of the user, the electronic device needs to determine whether the user has performed intense exercise before the measurement, and needs to determine an activity intensity of the user. A larger value of the activity intensity indicates more intense exercise of the user. A smaller value of the activity intensity indicates lower exercise intensity of the user. If an activity intensity of the user at a moment is greater than a preset activity threshold, it may be identified that the user is in an activity state, and the activity state may be caused by intense exercise of the user. To ensure that the user is in a stable state during blood pressure measurement, the electronic device obtains an activity intensity within preset detection duration before a moment at which the measurement operation is received, to detect whether the user is in intense exercise, namely, the active state, and determines whether rest time of the user is sufficient for blood pressure measurement when the user is in the active state.

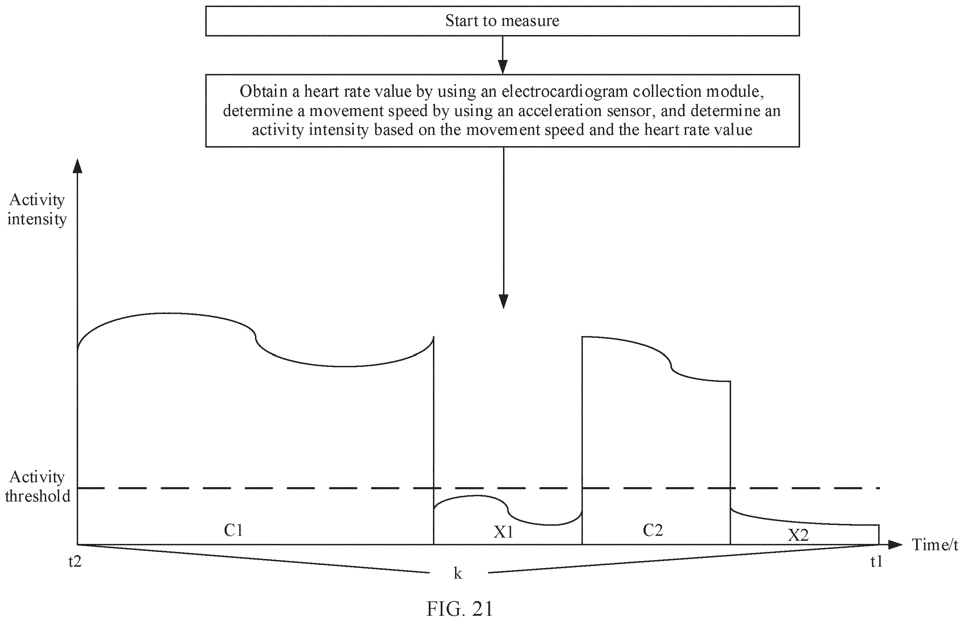

For example, FIG. 21 is a schematic diagram of time period division according to an embodiment of this application. As shown FIG. 21, an electronic device receives, at a moment t1, a measurement operation initiated by a user. In this case, the electronic device obtains an activity intensity within preset detection duration before the moment t1, for example, an activity intensity between t2 and t1. For example, if the preset detection duration is k, a time difference between t1 and t2 is k.

In a possible implementation, the activity intensity is obtained through calculation based on a heart rate value and a movement speed of the user. A higher heart rate value of the user indicates a larger value of a corresponding activity intensity. A higher movement speed of the user indicates a larger value of a corresponding activity intensity.

In this embodiment of this application, the activity intensity of the user within the preset detection duration before the blood pressure measurement is obtained, and the activity intensity is used as the first feature data, to determine whether the user performs intense exercise before the measurement, to ensure that blood pressure measurement is performed on the user in a stable state. In this way, accuracy of the blood pressure measurement can be improved.

S2002: Determine an abnormality type of the wearable device and/or the user based on the first feature data.

A specific implementation of S2002 in this embodiment is the same as that of S202 in Embodiment 1. For a specific description, refer to the related description of S202. Details are not described herein again.

Further, in another embodiment of this application, S2002 specifically includes S2002.1 to S2002.6. A specific description is as follows.

S2002.1: Divide the preset detection duration into an active time period and an inactive time period based on a preset activity threshold, where an activity intensity in the active time period is greater than or equal to the activity threshold, and an activity intensity in the inactive time period is less than the activity threshold.

In this embodiment, the electronic device may be preset with an activity threshold. The activity threshold is used to distinguish the active time period from the inactive time period. The activity threshold may be preconfigured in a system, or may be calculated based on a preset algorithm after data collected by the wearable device in a daily life process of the user is collected. A manner of determining the activity threshold is not limited herein.

In this embodiment, the electronic device may classify each time period within the preset detection duration based on the activity threshold, classify a time period in which a value of the activity intensity is greater than or equal to the activity threshold as the active time period, and classify a time period in which a value of the activity intensity is less than the activity threshold as the inactive time period.

Optionally, the preset detection duration includes at least one inactive time period. For example, if the user does not perform intense exercise before detection, the preset detection duration may include only one inactive time period, and include no active time period. Optionally, the preset detection duration includes at least one active time period. For example, if the user just completes exercise such as running before detection, the preset detection duration may include only one active time period.

For example, still refer to FIG. 21. A dashed line represents the activity threshold. Therefore, an activity intensity at each moment within the preset detection duration k may be compared with the activity threshold, to obtain a plurality of different time periods. C1 and C2 are active time periods, and an activity intensity in the active time period is greater than or equal to the activity threshold. X1 and X2 are inactive time periods, and an activity intensity in the inactive time period is less than the activity threshold.

S2002.2: Accumulate an integral of an activity intensity in each active time period, to determine a total activity amount of the user within the preset detection duration.

S2002.3: Determine expected rest duration of the user based on the total activity amount.

In this embodiment, the electronic device may determine the total activity amount of the user based on an activity intensity in all the active time periods. A manner of calculating the total activity amount may be: performing integration on an activity intensity in a time dimension, using a value obtained through integration as an activity amount corresponding to the active time period, and adding activity amounts in all active time periods to obtain, through calculation, the total activity amount within the preset detection duration. In a possible implementation, the electronic device may determine, based on a time difference between each active time period and a start moment, a weighted weight corresponding to the active time period. A longer time difference between the active time period and the start moment indicates a smaller corresponding weighted weight. On the contrary, a shorter time difference between the active time period and the start moment indicates a larger corresponding weighted weight. For example, the preset detection duration shown in FIG. 21 is used as an example, and the total activity amount may be specifically expressed as:

$$ActivityLv = Weight_{C1} * Activity_{C1} + Weight_{C2} * Activity_{C2}$$

$ActivityLv$ is the total activity amount, $Activity_{C1}$ is an activity amount in the time period C1, $Weight_{C1}$ is a weighted weight corresponding to the time period C1, $Activity_{C2}$ is an activity amount in the time period C2, and $Weight_{C2}$ is a weighted weight corresponding to the time period C2. Because the time period C2 is close to the start moment, $Weight_{C2}$ is greater than $Weight_{C1}$.

In this embodiment, a corresponding rest time conversion coefficient is set in the electronic device, and duration during which the user is expected to have a rest (which may also be the expected rest duration) of the user may be calculated by multiplying the total activity amount of the user by the conversion coefficient, that is, the expected rest duration may be expressed as:

$$\xi_{rest} = \gamma \sum_{Ci=1}^{n} Weight_{Ci} * Activity_{Ci}$$

$\xi_{rest}$ is the expected rest duration; $\gamma$ is the rest time conversion coefficient; $Activity_{Ci}$ is an activity amount in a $Ci^{th}$ active time period; $Weight_{Ci}$ is a weighted weight of the $Ci^{th}$ active time period, and certainly, the weighted weight may be 1; and n is a total quantity of active time periods.

S2002.4: Determine rested duration of the user based on a type of a time period to which the start moment belongs.

In this embodiment, the time period type includes an active time period and an inactive time period. If the type of the time period to which the start moment belongs is the active time period, the rested duration of the user is 0; or if the type of the time period to which the start moment belongs is the inactive time period, the duration of the inactive time period to which the start moment belongs is used as the rested duration of the user.

S2002.5: Determine whether the rested duration is less than the expected rest duration.

If the rest duration is less than the expected rest duration, an operation of S2002.6 is performed. On the contrary, if the rested duration is greater than or equal to the expected rest duration, it may be identified that the user is in a state in which the measurement condition is met, or the operation of S1001.1 in Embodiment 2 may be performed, to determine whether the wearable device is worn too loosely or too tightly, or the airbag leaks air.

S2002.6: If the rested duration is less than the expected rest duration, determine that the abnormality type of the user is a fourth abnormality type, where the fourth abnormality type indicates that the user is in a rest insufficient state.

In this embodiment, the electronic device may compare the rested duration with the expected rest duration, to determine whether the user has taken a full rest and is suitable for blood pressure measurement. Still refer to FIG. 21. The foregoing inactive time periods are X1 and X2, and an end moment of X2 is a start moment at which a measurement operation of the user is received, that is, X2 is a time period to which the start moment belongs, and the time period is the inactive time period. In this case, duration corresponding to X2 is used as rested duration of the user, and the rested duration is compared with the expected rest duration. If the expected rest duration is greater than or equal to the expected rest duration, it is identified that the user has taken a full rest, and the air pump is enabled to inflate the airbag. Then, comparison with an abnormality feature of another abnormality type may be performed, to further determine a measurement state of the wearable device. If the latest rested duration is less than the expected rest duration, it indicates that the user is not fully rested, and the user is not suitable for blood pressure measurement. In this case, the electronic device identifies that the measurement state of the user is a first state, and an abnormality type of the first state is the fourth abnormality type.

In this embodiment of this application, an active time period and an inactive time period of a user are identified, and expected rest duration of the user is determined, to determine whether the user has taken a full rest for blood pressure measurement, so that whether the user is suitable for blood pressure measurement can be determined based on an activity degree of the user. In this way, accuracy of a blood pressure measurement process is improved.

S2004: Output prompt information associated with the abnormality type.

A specific implementation of S2004 in this embodiment is the same as that of S203 in Embodiment 1. For a specific description, refer to the related description of S203. Details are not described herein again.

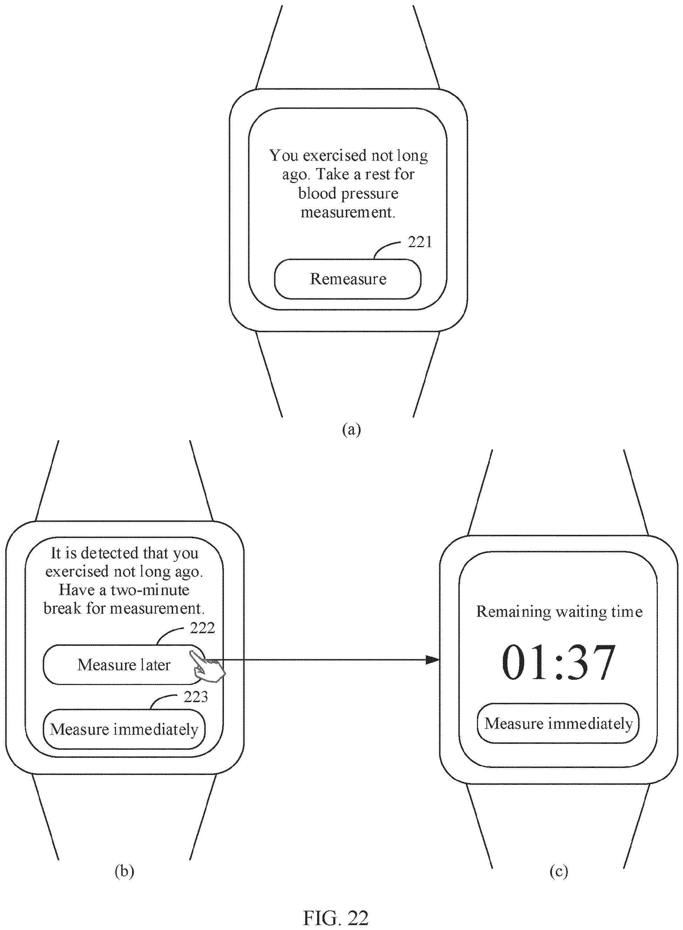

In a possible implementation, the electronic device may output prompt information that prompts the user to take a rest. For example, FIG. 22 is a schematic diagram of outputting prompt information according to an embodiment of this application. As shown in (a) in FIG. 22, the electronic device may output prompt information that prompts the user to take a rest, for example, "You exercised not long ago. Take a rest for blood pressure measurement". The user may take a rest based on the prompt information, and is remeasured after the rest. The prompt interface further includes a Remeasure control 221. After the rest, the user may tap the Remeasure control 221 to measure again.

Further, in another embodiment of this application, before the outputting prompt information associated with the abnormality type, the method further includes S2003. Correspondingly, prompt information associated with the abnormality type is output in S2004.1.

S2003: Determine required rest duration of the user based on a time difference between the expected rest duration and the rested duration.

S2004.1: Output fourth prompt information, where the fourth prompt information is used to prompt the user with the required rest duration.

In this embodiment, after detecting that the user does not take a full rest, the electronic device may further display, in the prompt information, the required rest duration of the user. The required rest duration is specifically a time difference between the expected rest duration and the rested duration. Refer to FIG. 21. The required rest duration is specifically a time difference between $\xi_{rest}$ and X2. When generating the prompt information, the electronic device may display the required rest duration, so that the user determines specific required rest duration. As shown in (b) in FIG. 22, content displayed in the foregoing prompt information is "It is detected that you exercised not long ago. Have a two-minute break for measurement.". Certainly, to improve readability, how long ago did you exercise may be further described, for example, "It is detected that you exercised three minutes ago. Have a two-minute break for measurement." Similarly, the foregoing prompt information is further configured with a Remeasure control, including a Later measurement control 222, and an Immediate measurement control 223. The Immediate measurement control 223 indicates that the user does not have a rest, but is directly measured immediately. This solution may introduce a measurement error, but the user agrees with the introduction of the error. The Later measurement control 222 indicates that the user agrees to be measured after waiting for specified duration. In this case, the wearable device may be configured with a timer and automatically performs a detection operation without a user's tap again when detecting that a count value of the timer matches the required rest duration. For another example, the electronic device may further generate a countdown page, as shown in (c) in FIG. 22, to count down the required rest duration. When a countdown value is 0, the measurement operation is performed.

In a possible implementation, if the user performs intense exercise within the required rest duration, and it is detected that time in which an activity intensity is greater than an activity threshold exists within the required rest duration, the required rest duration may be prolonged, and corresponding prompt information is regenerated to prompt the user to have a prolonged rest. For a calculation manner of the prolonged rest, refer to related descriptions of S2002.1 to S2002.5. Details are not described herein again.

In this embodiment of this application, the required rest duration of the user is displayed in the prompt information, so that the user can conveniently determine specific required rest time, thereby improving readability and guidance of the prompt information.

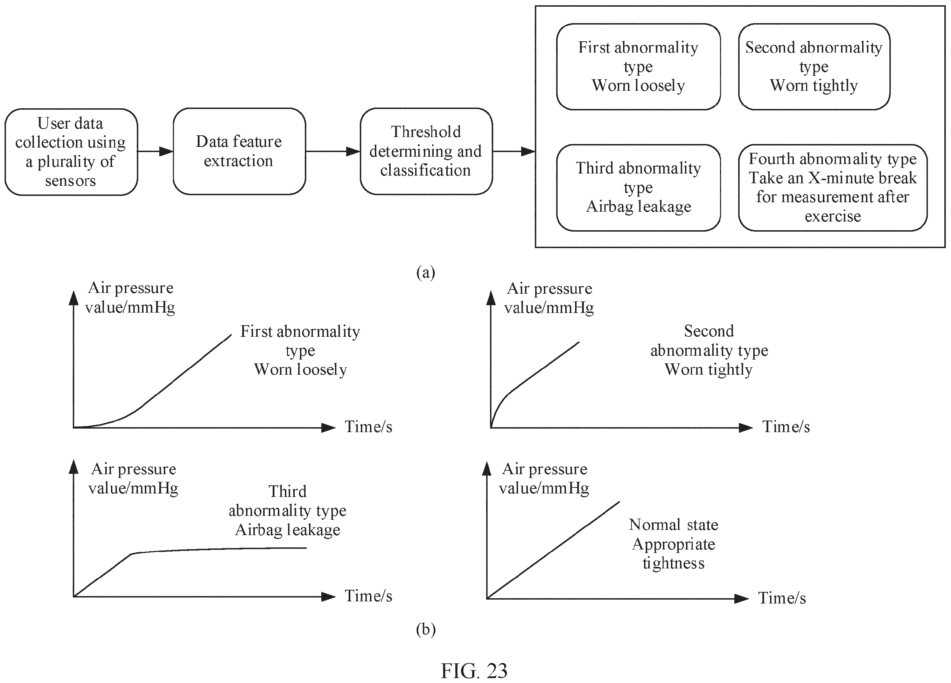

In conclusion, with reference to Embodiment 2 and Embodiment 3, the foregoing abnormality types specifically include four types. For example, FIG. 23 is a schematic diagram of abnormality type classification according to an embodiment of this application. As shown in (a) in FIG. 23, the wearable device may obtain user data of a user by using a built-in sensor, extract features of the user data to obtain first feature data, and perform threshold determining on the first feature data and an abnormality corresponding to each abnormality type, to classify abnormality types of a first measurement type, which are: a first abnormality type (worn loosely), a second abnormality type (worn tightly), a third abnormality type (airbag leakage), and a fourth abnormality type (take an X-minute break for measurement after exercise). Correspondingly, for air pressure change curves of the first abnormality type to the third abnormality type and the normal state, refer to (b) in FIG. 23.

Embodiment 4

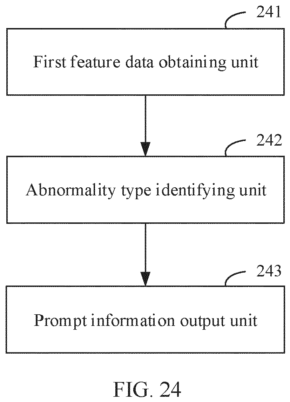

Corresponding to the prompt method in the foregoing embodiments, FIG. 24 is a block diagram of a structure of a prompt apparatus according to an embodiment of this application. For ease of description, only parts related to embodiments of this application are shown.

Refer to FIG. 24. The prompt apparatus is used in a wearable device. The wearable device includes an inflatable airbag, and the prompt apparatus includes:

a first feature data obtaining unit 241, configured to obtain first feature data of a user in response to a measurement operation, where the first feature data includes an activity intensity of the user and/or an air pressure value, and the air pressure value includes an air pressure value in the airbag when the user wears the wearable device to measure blood pressure;
an abnormality type identifying unit 242, configured to determine an abnormality type of the wearable device and/or the user based on the first feature data, where the abnormality type indicates that the wearable device and/or the user are/is currently in a state in which a measurement condition is not met; and
a prompt information output unit 243, configured to output prompt information associated with the abnormality type, where the prompt information is used to prompt the user to adjust the current state.

Optionally, the first feature data obtaining unit 241 includes:
an airbag inflation unit, configured to: control the airbag to be inflated in response to the measurement operation, collect an air pressure value in the airbag in an inflation process, and use the collected air pressure value as the first feature data.

Optionally, the abnormality type identifying unit 242 includes:

an air pressure change curve generation unit, configured to generate an air pressure change curve based on the air pressure value and airbag inflation time corresponding to each air pressure value; and
a measurement state classifying unit, configured to determine the abnormality type of the wearable device based on the air pressure change curve.

Optionally, the measurement state classifying unit includes:

a slope rise duration determining unit, configured to: if a first air pressure value collected at a first moment is less than or equal to a preset air pressure threshold, determine whether a corresponding moment at which a slope of the air pressure change curve increases to a preset slope is later than a second moment; and
a looseness abnormality identifying unit, configured to: if the corresponding moment at which the slope of the air pressure change curve increases to the preset slope is later than the second moment, determine that the wearable device is of a first abnormality type, where the first abnormality type indicates that wearing tightness of the wearable device is in a loose state, and the second moment is later than the first moment.

Optionally, the prompt apparatus further includes:
a to-be-tightened grid quantity determining unit, configured to determine, based on the corresponding moment at which the slope of the air pressure change curve increases to the preset slope, a quantity of to-be-tightened grids of a strap that need to be adjusted.
The prompt information output unit 243 includes:
a tightening prompt unit, configured to output first prompt information, where the first prompt information is used to prompt the user to tighten the strap of the wearable device by a specified quantity of grids, and the specified quantity of grids is the quantity of to-be-tightened grids of the strap.
Optionally, the measurement state classifying unit includes:
an over-tightening abnormality identifying unit, configured to: if a first air pressure value collected at a first moment is greater than a preset air pressure threshold, determine that the wearable device is of a second abnormality type, where the second abnormality type indicates that wearing tightness of the wearable device is in a tight state.
Optionally, the prompt apparatus further includes:

a second air pressure value determining unit, configured to determine a corresponding second air pressure value when a slope of the air pressure change curve decreases to a preset slope; and

a to-be-loosened grid quantity determining unit, configured to determine, based on the second air pressure value, a quantity of to-be-loosened grids of a strap that need to be adjusted.

The prompt information output unit 243 includes:

a loosening prompt unit, configured to output second prompt information, where the second prompt information is used to prompt the user to loosen the strap of the wearable device by a specified quantity of grids, and the specified quantity of grids is the quantity of to-be-loosened grids of the strap.

Optionally, the measurement state classifying unit includes:

an air leakage abnormality identifying unit, configured to: if a slope of the air pressure change curve is less than a preset slope at a preset third moment, determine that the abnormality type of the wearable device is a third abnormality type, where the third abnormality type indicates that the airbag is in an air leakage state.

In a possible implementation of the second aspect, the prompt information output unit 243 includes:

a send-for-repair prompt unit, configured to output third prompt information, where the third prompt information is used to prompt the user to send the wearable device for repair.

Optionally, the first feature data obtaining unit 241 includes:

an activity intensity determining unit, configured to: obtain, by using a moment at which the measurement operation is detected as a start moment, an activity intensity of the user within preset detection duration before the start moment, and use the activity intensity within the preset detection duration as the first feature data.

Optionally, the abnormality type identifying unit 242 includes:

an active time period division unit, configured to divide the preset detection duration into an active time period and an inactive time period based on a preset activity threshold, where an activity intensity in the active time period is greater than or equal to the activity threshold, and an activity intensity in the inactive time period is less than the activity threshold;

a total activity amount calculation unit, configured to accumulate an integral of an activity intensity in each active time period, to determine a total activity amount of the user within the preset detection duration;

an expected rest duration calculation unit, configured to determine expected rest duration of the user based on the total activity amount;

a rested duration determining unit, configured to determine rested duration of the user based on a type of a time period to which the start moment belongs; and

a rest abnormality identifying unit, configured to: if the rested duration is less than the expected rest duration, determine that the abnormality type of the user is a fourth abnormality type, where the fourth abnormality type indicates that the user is in a rest insufficient state.

Optionally, the prompt apparatus further includes:

a required rest duration determining unit, configured to determine required rest duration of the user based on a time difference between the expected rest duration and the rested duration.

The prompt information output unit 243 includes:

a rest prompt unit, configured to output fourth prompt information, where the fourth prompt information is used to prompt the user with the required rest duration.

Optionally, the wearable device includes a heart rate collection module and an acceleration sensor. The activity intensity is calculated based on a heart rate value obtained by the heart rate collection module and a movement speed determined by the acceleration sensor.

Optionally, the prompt apparatus further includes:

a remeasurement response unit, configured to obtain second feature data of the user in response to a remeasurement operation that is fed back by the user based on the prompt information; and

a measurement result output unit, configured to: if it is determined, based on the second feature data, that statuses of the wearable device and the user both meet the measurement condition, generate a measurement result based on the second feature data.

Therefore, according to the wearable device-based prompt apparatus provided in this embodiment of this application, when the measurement operation initiated by the user is received, the first feature data of the user may also be obtained by using the wearable device, and whether the user is currently in a state in which a measurement task can be completed is determined based on the collected first feature data. If the user is currently in a state in which a measurement task cannot be completed, namely, an abnormal state, an abnormality type corresponding to the user and/or the wearable device is determined based on the first feature data, and prompt information corresponding to the abnormality type is generated. The user may adjust the current measurement state based on the output prompt information, so that an adjusted state meets a condition for completing the measurement task, to automatically identify and prompt an abnormality in a measurement process. Compared with an existing wearable device-based measurement technology, in this embodiment, before the measurement result is output, whether the current measurement state meets a measurement condition may be determined based on the collected first feature data. If the current measurement state does not meet the measurement condition, the measurement task is not continued, but prompt information associated with the current measurement state is output. In this way, the user can discover in time that the current state does not meet a measurement requirement, so that an abnormal state is indicated in time, and a case in which a measurement result is inaccurate because a measurement state does not meet the condition is avoided. In this way, a measurement success rate is increased.

The wearable device-based prompt method provided in the embodiments of this application may be applied to electronic devices such as a mobile phone, a tablet computer, a wearable device, a vehicle-mounted device, an augmented reality (augmented reality, AR)/virtual reality (virtual reality, VR) device, a notebook computer, an ultra-mobile personal computer (ultra-mobile personal computer, UMPC), a netbook, and a personal digital assistant (personal digital assistant, PDA). Particularly, the wearable device-based prompt method may be applied to a wearable device that can collect user feature information, or another electronic device connected to the wearable device, for example, if a smartphone establishes a wireless connection to a wearable device, the foregoing prompt method may be executed on the smartphone, and corresponding prompt information is output.

For example, the electronic device may be a station (STATION, ST) in a WLAN, or may be a cellular phone, a cordless phone, a session initiation protocol (Session Initiation Protocol, SIP) phone, or a wireless local loop (Wireless Local Loop, WLL) station, a personal digital assistant (Personal Digital Assistant, PDA) device, a handheld device having a wireless communication function, a computing device or another processing device connected to a wireless modem, a computer, a laptop computer, a handheld communication device, a handheld computing device, and/or another device for performing communication in a wireless system, and a next-generation communication system, for example, a mobile terminal in a 5G network or a mobile terminal in a future evolved public land mobile network (Public Land Mobile Network, PLMN) network.

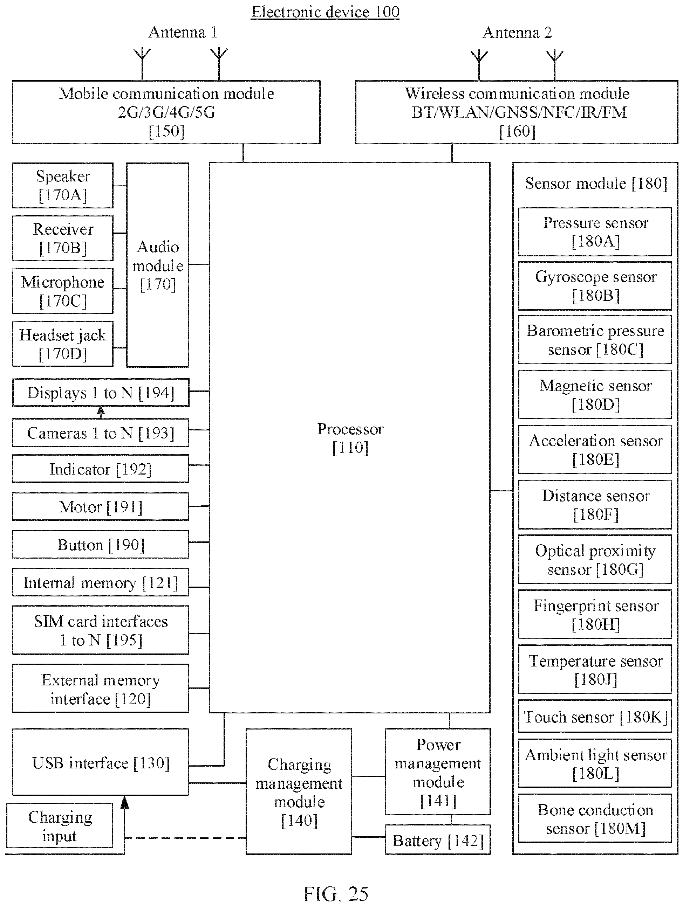

FIG. 25 is a schematic diagram of a structure of an electronic device 100.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache memory. The memory may store instructions or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a bidirectional synchronous serial bus, including a serial data line (serial data line, SDA) and a serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be used to perform audio communication, and sample, quantize, and code an analog signal. In some embodiments, the audio module 170 may be coupled to the wireless communication module 160 through a PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be used for audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral component like the display 194 or the camera 193. The MIPI interface includes a camera serial interface (camera serial interface, CSI), a display serial interface (display serial interface, DSI), and the like. In some embodiments, the processor 110 communicates with the camera 193 through the CSI, to implement a photographing function of the electronic device 100. The processor 110 communicates with the display 194 through the DSI interface, to implement a display function of the electronic device 100.

The GPIO interface may be configured by software. The GPIO interface may be configured for control signals or data signals. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB interface 130 is an interface that conforms to a USB standard specification, and may be specifically a mini USB interface, a micro USB interface, a USB type-C interface, or the like. The USB interface 130 may be configured to connect to a charger to charge the electronic device 100, or may be configured to exchange data between the electronic device 100 and a peripheral device, or may be configured to connect to a headset for playing audio through the headset. The interface may be further configured to connect to another electronic device like an AR device.

It can be understood that an interface connection relationship between modules illustrated in this embodiment of this application is merely an illustrative description, and does not constitute a limitation on a structure of the electronic device 100. In some other embodiments of this application, the electronic device 100 may alternatively use an interface connection manner different from that in the foregoing embodiment, or use a combination of a plurality of interface connection manners.

The charging management module 140 is configured to receive a charging input from a charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 100. The charging management module 140 supplies power to the electronic device through the power management module 141 while charging the battery 142.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive electromagnetic wave signals. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a solution of wireless communication that is applied to the electronic device 100 and that includes a 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by an audio device (which is not limited to the speaker 170A, the receiver 170B, or the like), or displays an image or a video by the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that is applied to the electronic device 100, and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processor module. The wireless communication module 160 receives an electromagnetic wave by the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 100 are coupled, and the antenna 2 and the wireless communication module 160 in the electronic device 100 are coupled, so that the electronic device 100 can communicate with a network and another device by using a wireless communication technology. The wireless communication technology may include a global system for mobile communication (global system for mobile communication, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (beidou navigation satellite system, BDS), a quasi-zenith satellite system (quasi-zenith satellite system, QZSS), and/or a satellite based augmentation system (satellite based augmentation system, SBAS).

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information. The display 194 may specifically display a generated detection report, so that a user can view the detection report by using the display 194.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device 100 may include one or N displays 194, where N is a positive integer greater than 1. The display 194 may include a touch panel and another input device.

The electronic device 100 may implement a photographing function through the ISP, the camera 193, the video codec, the GPU, the display 194, the application processor and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transformation on frequency energy.

The video codec is configured to compress or decompress a digital video. The electronic device 100 may support one or more video codecs. In this way, the electronic device 100 may play back or record videos in a plurality of coding formats, for example, moving picture experts group (moving picture experts group, MPEG)-1, MPEG-2, MPEG-3, and MPEG-4.

The NPU is a neural-network (neural-network, NN) computing processor, and simulates a biological neural network structure like a transmission mode between neurons in a human brain to perform rapid processing on input information, and can perform continuous self-learning. Applications such as intelligent cognition of the electronic device 100 may be implemented through the NPU, for example, image recognition, facial recognition, speech recognition, and text understanding.

The external memory interface 120 may be used to connect to an external storage card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a voice playing function or an image playing function), and the like. The data storage area may store data (such as audio data and an address book) created during use of the electronic device 100, and the like. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications and data processing of the electronic device 100.

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to code and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A. Particularly, the speaker 170A may be configured to output prompt information, to notify a user of a part that needs to be in contact with an electronic scale.

The receiver 170B, also referred to as an "earpiece", is configured to convert an audio electrical signal into a sound signal. When a call is answered or speech information is received through the electronic device 100, the receiver 170B may be put close to a human ear to listen to a voice.

The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, to implement a directional recording function and the like.

The headset jack 170D is configured to connect to a wired headset. The headset jack 170D may be a USB interface 130, or may be a 3.5 mm open mobile terminal platform (open mobile terminal platform, OMTP) standard interface or cellular telecommunication industry association of the USA (cellular telecommunication industry association of the USA, CTIA) standard interface.

The pressure sensor 180A is configured to sense a pressure signal, and can convert the pressure signal into an electrical signal. In some embodiments, the pressure sensor 180A may be disposed on the display 194. For example, the electronic device may obtain weight of the user by using the pressure sensor 180A. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch position based on a detection signal of the pressure sensor 180A. In some embodiments, touch operations that are performed in a same touch position but have different touch operation intensity may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating a new SMS message is performed.

The gyroscope sensor 180B may be configured to determine a moving posture of the electronic device 100. In some embodiments, an angular velocity of the electronic device 100 around three axes (namely, axes x, y, and z) may be determined through the gyroscope sensor 180B. The gyroscope sensor 180B may be configured to implement image stabilization during photographing. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a clamshell phone, the electronic device 100 may detect opening and closing of a flip cover based on the magnetic sensor 180D. Further, a feature like automatic unlocking of the flip cover is set based on a detected opening or closing state of the leather case or a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor 180E may be further configured to identify a posture of the electronic device, and is used in an application like switching between a landscape mode and a portrait mode or a pedometer.

The distance sensor 180F is configured to measure a distance. The electronic device 100 may measure the distance in an infrared manner or a laser manner. In some embodiments, in a photographing scenario, the electronic device 100 may measure a distance through the distance sensor 180F to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that the user holds the electronic device 100 close to an ear for a call, to automatically turn off a screen for power saving. The optical proximity sensor 180G may be further configured to automatically unlock and lock the screen in a leather cover mode and a pocket mode.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy through the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142 to prevent the electronic device 100 from being shut down abnormally due to a low temperature. In some other embodiments, when the temperature is lower than still another threshold, the electronic device 100 boosts an output voltage of the battery 142 to avoid abnormality shutdown caused by a low temperature.

The touch sensor 180K is also referred to as a "touch component". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor 180K may also be disposed on a surface of the electronic device 100 at a position different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a body pulse to receive a blood pressure beating signal. In some embodiments, the bone conduction sensor 180M may also be disposed in the headset, to obtain a bone conduction headset. The audio module 170 may obtain a speech signal through parsing based on the vibration signal that is of the vibration bone of the vocal-cord part and that is obtained by the bone conduction sensor 180M, to implement a speech function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a key input, and generate a key signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playback) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may be compatible with different types of SIM cards. The SIM card interface 195 is also compatible with an external storage card. The electronic device 100 interacts with a network through the SIM card, to implement functions such as conversation and data communication. In some embodiments, the electronic device 100 uses an eSIM, that is, an embedded SIM card. The eSIM card may be embedded into the electronic device 100, and cannot be separated from the electronic device 100.

A software system of the electronic device 100 may use a layered architecture, an event-driven architecture, a microkernel architecture, a micro service architecture, or a cloud architecture. In this embodiment of this application, an Android system with a layered architecture is used as an example to describe a software structure of the electronic device 100.

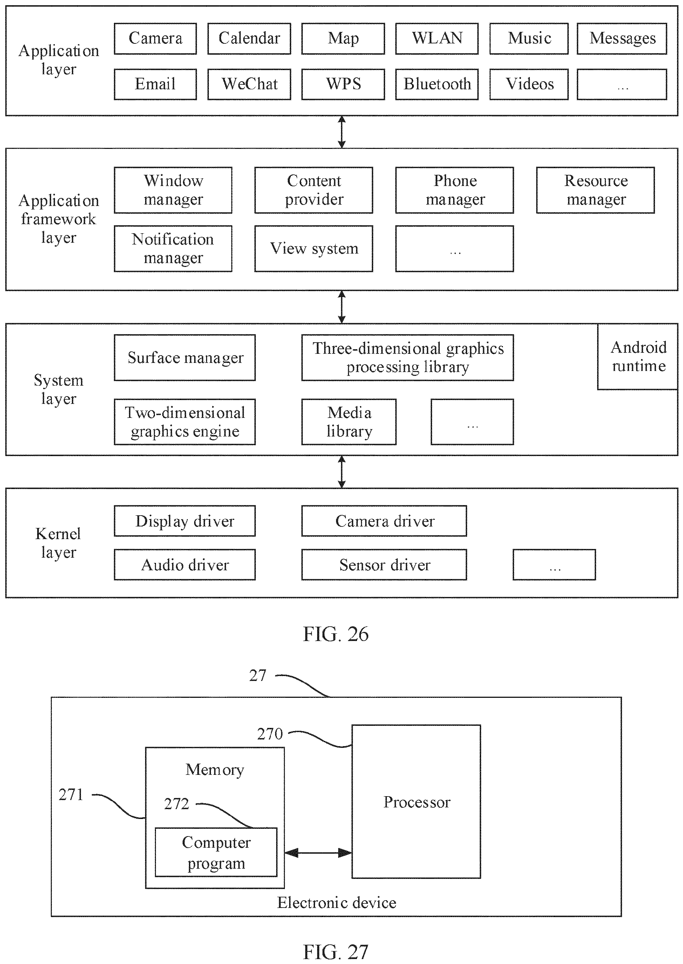

FIG. 26 is a block diagram of a software structure of an electronic device according to an embodiment of this application.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 26, an application package may include applications such as Camera, Gallery, Calendar, Phone, Map, Navigation, WLAN, Bluetooth, Music, Videos, and Messages.

The application framework layer provides an application programming interface (application programming interface, API) and a programming framework for an application at the application layer. The application framework layer includes some predefined functions.

As shown in FIG. 26, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and bookmarks, an address book, and the like.

The view system includes visual controls such as a control for displaying a text and a control for displaying an image. The view system may be configured to construct an application. A display interface may include one or more views. For example, a display interface including an SMS message notification icon may include a text display view and an image display view.

The phone manager is configured to provide a communication function of the electronic device, for example, management of a call status (including answering, declining, or the like).

The resource manager provides various resources such as a localized character string, an icon, an image, a layout file, and a video file for an application.

The notification manager enables an application to display notification information in a status bar, and may be configured to convey a notification message. The notification manager may automatically disappear after a short pause without requiring a user interaction. For example, the notification manager is configured to notify download completion, give a message notification, and the like. The notification manager may alternatively be a notification that appears in a top status bar of the system in a form of a graph or a scroll bar text, for example, a notification of an application that is run on a background, or may be a notification that appears on the screen in a form of a dialog window. For example, text information is displayed in the status bar, an announcement is given, the electronic device vibrates, or the indicator light blinks.

The Android runtime includes a kernel library and a virtual machine. The Android runtime is responsible for scheduling and management of the Android system.

The kernel library includes two parts: a function that needs to be called in Java language and a kernel library of Android.

The application layer and the application framework layer run on the virtual machine. The virtual machine executes java files of the application layer and the application framework layer as binary files. The virtual machine is configured to implement functions such as object lifecycle management, stack management, thread management, security and abnormality management, and garbage collection.

The system layer may include a plurality of functional modules, For example, the functional modules include a surface manager (surface manager), a media library (Media Library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, and static image files. The media library may support a plurality of audio and video encoding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working process of software and hardware of the electronic device 100 with reference to a photographing scenario.

When the touch sensor 180K receives a touch operation, a corresponding hardware interrupt is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event. An example in which the touch operation is a touch operation, and a control corresponding to the touch operation is a control of a camera application icon is used. The camera application invokes an interface of the application framework layer to enable the camera application, then enables the camera driver by invoking the kernel layer, and captures a static image or a video through the camera 193.

FIG. 27 is a schematic diagram of a structure of an electronic device according to an embodiment of this application. As shown in FIG. 27, the electronic device 27 in this embodiment includes: at least one processor 270 (FIG. 27 shows only one processor), a memory 271, and a computer program 272 that is stored in the memory 271 and that can run on the at least one processor 270. When executing the computer program 272, the processor 270 implements the steps in any one of the foregoing wearable device-based prompt method embodiments.

The electronic device 27 may be a computing device like a desktop computer, a notebook computer, a palmtop computer, or a cloud server. The electronic device may include but is not limited to the processor 270 and the memory 271. Persons skilled in the art may understand that FIG. 27 is merely an example of the electronic device 27, and does not constitute a limitation on the electronic device 27. The electronic device may include more or fewer components than those shown in the figure, or may combine some components, or may have different components. For example, the electronic device may further include an input/output device, a network access device, or the like.

The processor 270 may be a central processing unit (Central Processing Unit, CPU), or the processor 270 may be another general-purpose processor, a digital signal processor (Digital Signal Processor, DSP), an application specific integrated circuit (Application Specific Integrated Circuit, ASIC), a field-programmable gate array (Field-Programmable Gate Array, FPGA), or another programmable logic device, discrete gate or transistor logic device, discrete hardware component, or the like. The general-purpose processor may be a microprocessor, or the processor may be any conventional processor or the like.

In some embodiments, the memory 271 may be an internal storage unit of the electronic device 27, for example, a hard disk or a memory of the electronic device 27. In some other embodiments, the memory 271 may alternatively be an external storage device of the electronic device 27, for example, a plug-connected hard disk, a smart media card (Smart Media Card, SMC), a secure digital (Secure Digital, SD) card, or a flash card (Flash Card) that is disposed on the electronic device 27. Further, the memory 271 may further include both an internal storage unit of the electronic device 27 and an external storage device. The memory 271 is configured to store an operating system, an application, a bootloader (BootLoader), data, another program, and the like, for example, program code of the computer program. The memory 271 may be further configured to temporarily store data that has been output or that is to be output.

It should be noted that, content such as information exchange and execution processes between the foregoing apparatuses/units are based on a same concept as those in the method embodiments of this application. For specific functions and brought technical effects of the foregoing apparatuses/units, refer to the method embodiments. Details are not described herein again.

Persons skilled in the art may clearly understand that, for the purpose of convenient and brief description, division into the foregoing functional units and modules is merely used as an example for description. In an actual application, the foregoing functions may be allocated to different functional units and modules for implementation based on a requirement. In other words, an inner structure of the apparatus is divided into different functional units or modules, to implement all or some of the functions described above. Functional units and modules in embodiments may be integrated into one processing unit, or each of the units may exist alone physically, or two or more units may be integrated into one unit. The integrated unit may be implemented in a form of hardware, or may be implemented in a form of a software functional unit. In addition, specific names of the functional units and modules are merely for ease of distinguishing between the functional units and modules, but are not intended to limit the protection scope of this application. For a specific working process of the units or modules in the foregoing system, refer to a corresponding process in the method embodiments. Details are not described herein again.

An embodiment of this application further provides an electronic device. The electronic device includes: at least one processor, a memory, and a computer program that is stored in the memory and that is executable on the at least one processor. When executing the computer program, the processor implements the steps in any one of the foregoing method embodiments.

An embodiment of this application further provides a computer-readable storage medium. The computer-readable storage medium stores a computer program. When the computer program is executed by a processor, the steps in the foregoing method embodiments can be implemented.

An embodiment of this application provides a computer program product. When the computer program product runs on a mobile terminal, the steps in the foregoing method embodiments can be implemented by the mobile terminal.

When the integrated unit is implemented in the form of the software functional unit and sold or used as an independent product, the integrated unit may be stored in a computer-readable storage medium. Based on such an understanding, in this application, all or some of the procedures of the methods in the foregoing embodiments may be implemented by a computer program instructing related hardware. The computer program may be stored in a computer-readable storage medium. When the computer program is executed by a processor, the steps in the method embodiments can be implemented. The computer program includes computer program code, and the computer program code may be in a source code form, an object code form, an executable file, an intermediate form, or the like. The computer-readable medium may include at least any entity or apparatus that can carry the computer program code to a photographing apparatus/electronic device, a recording medium, a computer memory, a read-only memory (ROM, Read-Only Memory), a random access memory (RAM, Random Access Memory), an electrical carrier signal, a telecommunication signal, and a software distribution medium, for example, a USB flash drive, a removable hard disk, a magnetic disk, or an optical disk. In some jurisdictions, the computer-readable medium cannot be an electrical carrier signal or a telecommunication signal according to legislation and patent practices.

In the foregoing embodiments, the description of each embodiment has respective focuses. For a part that is not described in detail or recorded in an embodiment, refer to related descriptions in other embodiments.

Persons of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraint conditions of the technical solutions. Persons skilled in the art may use different methods to implement the described functions for each particular application, but it should not be considered that the implementation goes beyond the scope of this application.

In embodiments provided in this application, it should be understood that the disclosed apparatus/network device and method may be implemented in other manners. For example, the described apparatus/network device embodiment is merely an example. For example, division into the modules or units is merely logical function division and may be other division during actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

The foregoing embodiments are merely intended to describe the technical solutions of this application, but are not to limit this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the spirit and scope of the technical solutions of embodiments of this application, and these modifications and replacements shall fall within the protection scope of this application.

## Claims

1. A prompt method, applied to a wearable device, wherein the wearable device comprises an inflatable airbag, and the prompt method comprises:

   obtaining first feature data of a user in response to a measurement operation, wherein the first feature data comprises an activity intensity of the user and/or an air pressure value, and the air pressure value comprises an air pressure value in the airbag when the user wears the wearable device to measure blood pressure;
   determining an abnormality type of the wearable device and/or the user based on the first feature data, wherein the abnormality type indicates that the wearable device and/or the user are/is currently in a state in which a measurement condition is not met; and
   outputting prompt information associated with the abnormality type, wherein the prompt information is used to prompt the user to adjust the current state.

2. The prompt method according to claim 1, wherein the determining an abnormality type of the wearable device and/or the user based on the first feature data comprises:

generating an air pressure change curve based on the air pressure value and airbag inflation time corresponding to each air pressure value; and
determining the abnormality type of the wearable device based on the air pressure change curve.

3. The prompt method according to claim 2, wherein the determining the abnormality type of the wearable device based on the air pressure change curve comprises:

if a first air pressure value collected at a first moment is less than or equal to a preset air pressure threshold, determining whether a corresponding moment at which a slope of the air pressure change curve increases to a preset slope is later than a second moment; and
if the corresponding moment at which the slope of the air pressure change curve increases to the preset slope is later than the second moment, determining that the wearable device is of a first abnormality type, wherein the first abnormality type indicates that wearing tightness of the wearable device is in a loose state, and the second moment is later than the first moment.

4. The prompt method according to claim 3, wherein before the outputting prompt information associated with the abnormality type, the method further comprises:

determining, based on the corresponding moment at which the slope of the air pressure change curve increases to the preset slope, a quantity of to-be-tightened grids of a strap that need to be adjusted; and
the outputting prompt information associated with the abnormality type comprises:
outputting first prompt information, wherein the first prompt information is used to prompt the user to tighten the strap of the wearable device by a specified quantity of grids, and the specified quantity of grids is the quantity of to-be-tightened grids of the strap.

5. The prompt method according to claim 2, wherein the determining the abnormality type of the wearable device based on the air pressure change curve comprises:
if a first air pressure value collected at a first moment is greater than a preset air pressure threshold, determining that the wearable device is of a second abnormality type, wherein the second abnormality type indicates that wearing tightness of the wearable device is in a tight state.

6. The prompt method according to claim 5, wherein before the outputting prompt information associated with the abnormality type, the method further comprises:

determining a corresponding second air pressure value when a slope of the air pressure change curve decreases to a preset slope; and
determining, based on the second air pressure value, a quantity of to-be-loosened grids of a strap that need to be adjusted; and
the outputting prompt information associated with the abnormality type comprises:
outputting second prompt information, wherein the second prompt information is used to prompt the user to loosen the strap of the wearable device by a specified quantity of grids, and the specified quantity of grids is the quantity of to-be-loosened grids of the strap.

7. The prompt method according to claim 2, wherein the determining the abnormality type of the wearable device based on the air pressure change curve comprises:
if a slope of the air pressure change curve is less than a preset slope at a preset third moment, determining that the abnormality type of the wearable device is a third abnormality type, wherein the third abnormality type indicates that the airbag is in an air leakage state.

8. The prompt method according to claim 7, wherein the outputting prompt information associated with the abnormality type comprises:
outputting third prompt information, wherein the third prompt information is used to prompt the user to send the wearable device for repair.

9. The prompt method according to any one of claims 1 to 8, wherein the obtaining first feature data of a user in response to a measurement operation comprises:

obtaining, by using a moment at which the measurement operation is detected as a start moment, an activity intensity of the user within preset detection duration before the start moment, and using the activity intensity within the preset detection duration as the first feature data.

10. The prompt method according to claim 9, wherein the determining an abnormality type of the wearable device and/or the user based on the first feature data comprises:

dividing the preset detection duration into an active time period and an inactive time period based on a preset activity threshold, wherein an activity intensity in the active time period is greater than or equal to the activity threshold, and an activity intensity in the inactive time period is less than the activity threshold;
accumulating an integral of an activity intensity in each active time period, to determine a total activity amount of the user within the preset detection duration;
determining expected rest duration of the user based on the total activity amount;
determining rested duration of the user based on a type of a time period to which the start moment belongs; and
if the rested duration is less than the expected rest duration, determining that the abnormality type of the user is a fourth abnormality type, wherein the fourth abnormality type indicates that the user is in a rest insufficient state.

11. The prompt method according to claim 10, wherein before the outputting prompt information associated with the abnormality type, the method further comprises:

determining required rest duration of the user based on a time difference between the expected rest duration and the rested duration; and
the outputting prompt information associated with the abnormality type comprises:
outputting fourth prompt information, wherein the fourth prompt information is used to prompt the user with the required rest duration.

12. The prompt method according to any one of claims 9 to 11, wherein the wearable device comprises a heart rate collection module and an acceleration sensor, and the activity intensity is calculated based on a heart rate value obtained by the heart rate collection module and a movement speed determined by the acceleration sensor.

13. The prompt method according to any one of claims 1 to 12, wherein after the outputting prompt information associated with the abnormality type, the method further comprises:

obtaining second feature data of the user in response to a remeasurement operation that is fed back by the user based on the prompt information; and
if it is determined, based on the second feature data, that statuses of the wearable device and the user both meet the measurement condition, generating a measurement result based on the second feature data.

14. A prompt apparatus, used in a wearable device, wherein the wearable device comprises an inflatable airbag, and the prompt apparatus comprises:

a first feature data obtaining unit, configured to obtain first feature data of a user in response to a measurement operation, wherein the first feature data comprises an activity intensity of the user and/or an air pressure value, and the air pressure value comprises an air pressure value in the airbag when the user wears the wearable device to measure blood pressure;
an abnormality type identifying unit, configured to determine an abnormality type of the wearable device and/or the user based on the first feature data, wherein the abnormality type indicates that the wearable device and/or the user are/is currently in a state in which a measurement condition is not met; and
a prompt information output unit, configured to output prompt information associated with the abnormality type, wherein the prompt information is used to prompt the user to adjust the current state.

15. An electronic device, comprising a memory, a processor, and a computer program that is stored in the memory and that can be run on the processor, wherein when executing the computer program, the processor implements the method according to any one of claims 1 to 13.

16. A computer-readable storage medium, wherein the computer-readable storage medium stores a computer program, and when the computer program is executed by a processor, the method according to any one of claims 1 to 13 is implemented.

(a)

(b)

FIG. 1

S201

Obtain first feature data of a user in response to a measurement operation

S202

Determine an abnormality type of a wearable device and/or the user based on the first feature data

S203

Output prompt information associated with the abnormality type, where the prompt information is used to prompt the user to adjust a current state

S204

Obtain second feature data of the user by using the wearable device in response to a remeasurement operation that is fed back by the user based on the prompt information

S205

If it is determined, based on the second feature data, that statuses of the wearable device and the user both meet a measurement condition, generate a measurement result based on the second feature data

FIG. 2

(a)

(b)

FIG. 3

FIG. 4(a)

FIG. 4(b)

Smartwatch

Firmware update / 42

Appearance settings / 43

Blood pressure measurement / 44

FIG. 4(c)

FIG. 5(a)

FIG. 5(b)

FIG. 5(c)

FIG. 5(d)

FIG. 5(e)

FIG. 5(f)

The PPG module is
blocked.
Wipe the PPG module.

Remeasure

(a)

The PPG module is
blocked.
Wipe the PPG module.

PPG

Remeasure

(b)

The watch is worn too
loosely.
Tighten the strap.

Remeasure

(c)

The watch is worn too
loosely.
Tighten the strap
by one grid.

Remeasure

(d)

FIG. 6

Displayed on a
display module

Broadcast by
using a speaker

The watch is
worn too loosely

An indicator
light blinks

FIG. 7(a)

Broadcast by using a speaker

Prompt:
The wearable device is worn too loosely.
Tighten the strap.

| | | | |
|---|---|---|---|
| Clock | Calendar | Gallery | Notepad |
| Files | Email | Music | Calculator |
| Huawei video | Health | Weather | Browser |
| AI life | Settings | Recorder | AppGallery |
| Navigation | | | |
| Camera | Contacts | Phone | Messages |

Displayed in a pop-up box

FIG. 7(b)-1

5G 5G 📶 🔋 8:00

← Blood pressure measurement

Systolic blood pressure:
to be measured

Diastolic pressure:
to be measured

Abnormality:

The wearable device is worn too loosely.
Tighten the strap.

Remeasure

≡ Measurement
records

Measurement
settings

Displayed on an application

FIG. 7(b)-2

FIG. 7(c)

The watch is worn too loosely.
Tighten the strap buckle by one grid.

81

Remeasure

(a)

Systolic blood
pressure: 98 mmHg

Diastolic blood
pressure: 78 mmHg

Remeasure

Stop

(b)

FIG. 8

92

921

91

911 and 912

FIG. 9

S1001

Control an airbag to be inflated in response to a
measurement operation, and collect an air pressure
value in the airbag in an inflation process

S1002

Determine an abnormality type of a wearable device
and/or a user based on first feature data

S10021

Generate an air pressure change curve based on the air pressure value and
airbag inflation time corresponding to each air pressure value

S10022

Determine the abnormality type of the wearable device based on the air
pressure change curve

S10022.1

Obtain a first air pressure value that is fed
back by the airbag at a first moment

S10022.2

Is
the first air
pressure value
greater than an
air pressure
threshold?

Yes

No

S10022.4

Determine a first slope of the air pressure
change curve at a second moment

S10022.6

Generate a
measurement
result

Yes

S10022.5

Is
the first slope a
preset slope?

TO
FIG. 10B

TO
FIG. 10B

FIG. 10A

CONT.
FROM
FIG. 10A

CONT.
FROM
FIG. 10A

No

— S10022.7

Is
a second slope the
preset slope?

Yes

No

No

— S10022.8

Is
a third moment
reached?

Yes

S10022.9

S10022.10

S10022.3

| Determine the abnormality type as a first abnormality type | Determine the abnormality type as a third abnormality type | Determine the abnormality type as a second abnormality type |
|---|---|---|

— S1003.2

Determine a quantity
of to-be-tightened
grids of a strap that
need to be adjusted at
a corresponding
moment at which the
slope increases to the
preset slope

— S1003.1

Determine a quantity of
to-be-loosened grids of
a strap that need to be
adjusted based on a
second air pressure
value

— S1004

S1004.2

S1004.3

S1004.1

| Output first prompt information | Output third prompt information | Output second prompt information |
|---|---|---|

Output prompt information associated with the abnormality type

FIG. 10B

FIG. 11

FIG. 12

The strap is worn tightly. Loosen the strap buckle by two grids.

Remeasure

FIG. 13

Air pressure value/mmHg

100

Curve 1
Curve 2

T0  T1  Ta  T2  Tb  T3  T4  T5  Third moment

Time/s

FIG. 14

(a)

(b)

FIG. 15

The strap is worn loosely. Tighten the strap buckle by two grids.

Remeasure

FIG. 16

Air pressure value/mmHg

100

First moment

Second moment

Third moment

60

Time/s

FIG. 17

The airbag leaks air.
Send the airbag for
repair.

Remeasure

FIG. 18(a)

The airbag leaks air.
Send the airbag for
repair. Select a repair
center.

Repair center A

181

Repair center B

FIG. 18(b)

FIG. 18(c)

The airbag leaks air. Send
the airbag for repair.

Repair phone number ⎯⎯ 182

8888–8888

FIG. 18(d)

Calling

8888–8888
00:15

FIG. 18(e)

192

1921

1913

191

1911, 1912, 1914, and
1915

FIG. 19

S2001

Obtain, by using a moment at which a measurement operation is detected as a start moment, an activity intensity within preset detection duration before the start moment, and use the activity intensity within the preset detection duration as first feature data

S2002

Determine an abnormality type of a wearable device and/or a user based on the first feature data

S2002.1

Divide the preset detection duration into an active time period and an inactive time period based on a preset activity threshold

S2002.2

Accumulate an integral of an activity intensity in each active time period, to determine a total activity amount of the user within the preset detection duration

S2002.3

Determine expected rest duration of the user based on the total activity amount

S2002.4

Determine rested duration of the user based on a type of a time period to which the start moment belongs

S2002.5

Is the rested duration less than the expected rest duration?

No

Yes

S2002.6

Determine the abnormality type of the user as a fourth abnormality type

Go to step S1001

S2003

Determine required rest duration of the user based on a time difference between the expected rest duration and the rested duration

S2004

Output prompt information associated with the abnormality type

S2004.1

Output fourth prompt information, where the fourth prompt information is used to prompt the user with the required rest duration

FIG. 20

Start to measure

Obtain a heart rate value by using an electrocardiogram collection module,
determine a movement speed by using an acceleration sensor, and determine an
activity intensity based on the movement speed and the heart rate value

Activity
intensity

Activity
threshold

C1

X1

C2

X2

Time/t

t2

k

t1

FIG. 21

You exercised not long ago. Take a rest for blood pressure measurement.

221

Remeasure

(a)

It is detected that you exercised not long ago. Have a two-minute break for measurement.

222

Measure later

223

Measure immediately

(b)

Remaining waiting time

01:37

Measure immediately

(c)

FIG. 22

FIG. 23

241

First feature data obtaining unit

242

Abnormality type identifying unit

243

Prompt information output unit

FIG. 24

Electronic device 100

Antenna 1

Antenna 2

| Mobile communication module 2G/3G/4G/5G [150] | | Wireless communication module BT/WLAN/GNSS/NFC/IR/FM [160] |
|---|---|---|

Speaker [170A]

Receiver [170B]

Audio module [170]

Microphone [170C]

Headset jack [170D]

Displays 1 to N [194]

Cameras 1 to N [193]

Indicator [192]

Motor [191]

Button [190]

Internal memory [121]

SIM card interfaces 1 to N [195]

External memory interface [120]

USB interface [130]

Charging input

Charging management module [140]

Power management module [141]

Battery [142]

Processor [110]

Sensor module [180]

Pressure sensor [180A]

Gyroscope sensor [180B]

Barometric pressure sensor [180C]

Magnetic sensor [180D]

Acceleration sensor [180E]

Distance sensor [180F]

Optical proximity sensor [180G]

Fingerprint sensor [180H]

Temperature sensor [180J]

Touch sensor [180K]

Ambient light sensor [180L]

Bone conduction sensor [180M]

FIG. 25

| Application layer | Camera | Calendar | Map | WLAN | Music | Messages |
|---|---|---|---|---|---|---|
| | Email | WeChat | WPS | Bluetooth | Videos | ... |

| Application framework layer | Window manager | Content provider | Phone manager | Resource manager |
|---|---|---|---|---|
| | Notification manager | View system | ... | |

| System layer | Surface manager | Three-dimensional graphics processing library | Android runtime |
|---|---|---|---|
| | Two-dimensional graphics engine | Media library | ... | |

| Kernel layer | Display driver | Camera driver | |
|---|---|---|---|
| | Audio driver | Sensor driver | ... |

FIG. 26

27

270

271 Memory

272 Computer program

Processor

Electronic device

FIG. 27

## INTERNATIONAL SEARCH REPORT

| International application No. |
| --- |
| **PCT/CN2022/131955** |

**A. CLASSIFICATION OF SUBJECT MATTER**

A61B 5/00(2006.01)i; A61B 5/021(2006.01)i; A61B 5/0245(2006.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNPAT, CNKI, WPI, EPODOC: 可穿戴, 气囊, 气压, 压力, 活动, 运动, 调整, 松, 紧, 加速度, 心率, 调节, 异常, 表带, 斜率, 血压, 漏气, wearable, balloon, Sphygmomanometer, loose, pressure, air, acceleration, heart, rate, watchband, anormaly

**C. DOCUMENTS CONSIDERED TO BE RELEVANT**

| Category* | Citation of document, with indication, where appropriate, of the relevant passages | Relevant to claim No. |
| --- | --- | --- |
| X | CN 113520307 A (HUAWEI TECHNOLOGIES CO., LTD.) 22 October 2021 (2021-10-22) description, paragraphs 0006-0022 and paragraphs 99-107, and figure 1 | 1-16 |
| A | CN 106377402 A (SHENZHEN PUMP MEDICAL SYSTEM CO., LTD.) 08 February 2017 (2017-02-08) entire document | 1-16 |
| A | CN 112568884 A (HUAWEI DEVICE CO., LTD.) 30 March 2021 (2021-03-30) entire document | 1-16 |
| A | CN 112568887 A (SHENZHEN SHULIAN TIANXIA INTELLIGENT TECHNOLOGY CO., LTD.) 30 March 2021 (2021-03-30) entire document | 1-16 |
| A | CN 105045234 A (XI'AN JIAOTONG UNIVERSITY) 11 November 2015 (2015-11-11) entire document | 1-16 |
| A | CN 106073710 A (NINGBO LIXIN INFORMATION SCIENCE & TECHNOLOGY CO., LTD.) 09 November 2016 (2016-11-09) entire document | 1-16 |
| A | US 2016189051 A1 (MAHMOOD, Junayd fahim) 30 June 2016 (2016-06-30) entire document | 1-16 |

☐ Further documents are listed in the continuation of Box C. ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **27 December 2022** | **12 January 2023** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

| International application No. |
| --- |
| **PCT/CN2022/131955** |

| Patent document cited in search report | | | Publication date (day/month/year) | Patent family member(s) | | | Publication date (day/month/year) |
| --- | --- | --- | --- | --- | --- | --- | --- |
| CN | 113520307 | A | 22 October 2021 | None | | | |
| CN | 106377402 | A | 08 February 2017 | None | | | |
| CN | 112568884 | A | 30 March 2021 | None | | | |
| CN | 112568887 | A | 30 March 2021 | None | | | |
| CN | 105045234 | A | 11 November 2015 | WO | 2017008321 | A1 | 19 January 2017 |
| CN | 106073710 | A | 09 November 2016 | None | | | |
| US | 2016189051 | A1 | 30 June 2016 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111437864